# EUROPEAN PATENT SPECIFICATION

(11) **EP 4 388 311 B1**
(45) Date of publication and mention of the grant of the patent: **15.10.2025**
(21) Application number: 22797196.7
(22) Date of filing: 19.08.2022
(51) Int. Cl.: G01N 33/15, G01N 33/50, C12N 5/071, C07K 16/00

(54) **METHODS COMPRISING THERAPEUTIC COMPOUNDS AND IN VITRO MAMMALIAN SKIN**
VERFAHREN MIT THERAPEUTISCHEN VERBINDUNGEN UND IN-VITRO-SÄUGETIERHAUT
MÉTHODES FAISANT APPEL À DES COMPOSÉS THÉRAPEUTIQUES ET À UNE PEAU DE MAMMIFÈRE IN VITRO

(30) Priority: 20.08.2021 US 202163235371 P; 08.08.2022 US 202263370751 P
(43) Date of publication of application: 26.06.2024
(73) Proprietor: Amgen Inc., Thousand Oaks, California 91320 (US)
(72) Inventor: JOUBERT, Marisa, Thousand Oaks, California 91320-1799 (US); JOH, Nathan, H., Thousand Oaks, California 91320-1799 (US); TOKUDA, Joshua, M., Thousand Oaks, California 91320-1799 (US); FERBAS, John, Thousand Oaks, California 91320-1799 (US); XIE, Jiansong, Thousand Oaks, California 91320-1799 (US); XIANG, Dong, Thousand Oaks, California 91320-1799 (US)
(74) Representative: Dörries, Hans Ulrich
(86) International application number: PCT/US2022/075191
(87) International publication number: WO 2023/023633

(56) References cited:
- US-A1- 2020 400 652
- NG K W ET AL: "Development of an ex vivo human skin model for intradermal vaccination: Tissue viability and Langerhans cell behaviour", VACCINE, ELSEVIER, AMSTERDAM, NL, vol. 27, no. 43, 9 October 2009 (2009-10-09), pages 5948 - 5955, XP026643891, ISSN: 0264-410X, [retrieved on 20090811], DOI: 10.1016/J.VACCINE.2009.07.088
- BART DE WEVER ET AL: "Human Skin Models for Research Applications in Pharmacology and Toxicology: Introducing NativeSkin ® , the "Missing Link" Bridging Cell Culture and/or Reconstructed Skin Models and Human Clinical Testing", APPLIED IN VITRO TOXICOLOGY, vol. 1, no. 1, 11 February 2015 (2015-02-11), US, pages 26 - 32, XP055538973, ISSN: 2332-1512, DOI: 10.1089/aivt.2014.0010
- EBERLIN SAMARA ET AL: "The Ex Vivo Skin Model as an Alternative Tool for the Efficacy and Safety Evaluation of Topical Products", ATLA. ALTERNATIVES TO LABORATORY ANIMALS, vol. 48, no. 1, 4 June 2020 (2020-06-04), GB, pages 10 - 22, XP055982756, ISSN: 0261-1929, DOI: 10.1177/0261192920914193

## Description

### FIELD

Embodiments herein relate to methods of using *in vitro* mammalian skin for analysis, characterization, development, and/or selection of therapeutic compounds.

### BACKGROUND

Subcutaneous (SubQ) injection is a widely used administration route for pharmaceutical compositions comprising therapeutic compounds such as therapeutic proteins. However, aggregation and poor solubility can be major hurdles for therapeutic compound pharmaceutical composition manufacturing and delivery.

Using *in vitro* subcutaneous injections for testing pharmaceutical compositions on live skin is known from US 2020/400652 A1.

### SUMMARY

The invention relates to a method of selecting a condition of administration for a therapeutic compound for subcutaneous administration as defined in claim 1. Other aspects of the invention are described in the dependent claims.

For any of the methods of selecting a condition of administration for a therapeutic compound product for subcutaneous administration described herein, the condition of administration comprises at least one of formulation, concentration, volume, viscosity, a molecular attribute of said therapeutic compound, route of administration, and/or administration apparatus or method of using an administration apparatus (frequency, flow rate, parameter).

For any of the methods of selecting a condition of administration for a therapeutic compound product for subcutaneous administration described herein, the condition of administration may comprise an excipient, buffer, surfactant or stabilizer of a formulation.

For any of the methods of selecting a condition of administration for a therapeutic compound product for subcutaneous administration described herein, the physiochemical and/or clinical characteristic may comprise bioavailability, pharmacokinetic property, aggregation, precipitation, distribution, diffusion rate, retention and/or absorption.

For any of the methods of selecting a condition of administration for a therapeutic compound product for subcutaneous administration described herein, the therapeutic compound product may have acquired the physiochemical and/or clinical characteristic after said administering.

For any of the methods of selecting a condition of administration for a therapeutic compound product for subcutaneous administration described herein, the method may further comprise repeating the method for two or more different conditions of administration.

For any of the methods described herein, the molecular attribute may comprise at least one of acidic species, basic species, high molecular weight species, subvisible particle number, low molecular weight, middle molecular weight, glycosylation (such as non-glycosylated heavy chain or high mannose), non-heavy chain and light chain, deamidation, deamination, cyclization, oxidation, isomerization, fragmentation/clipping, N-terminal and C-terminal variants, reduced and partial species, folded structure, surface hydrophobicity, chemical modification, covalent bond, a C-terminal amino acid motif PARG, or a C-terminal amino acid motif PAR-Amide.

For any of the methods described herein, administering may comprise pressing a needle comprising a cannula to a surface of the dermal layer, thus inserting the needle in the interface of the live *in vitro* mammalian skin. The administering may further comprise disposing the therapeutic compound product in the subcutaneous space via the needle. In some methods, the needle is pressed against the dermal surface at an acute angle. In some methods, the needle comprises an opening in fluid communication with the cannula, wherein administering comprises orienting the opening toward the dermal layer. The detecting may comprise optical imaging through the dermal layer.

For any of the methods described herein, administering may comprise soaking, diffusion, or transdermal administration of the therapeutic compound product through the dermal layer and/or into the hypodermal layer.

For any of the methods described herein, the *in vitro* mammalian skin may further comprise a hypodermis that defines a subcutaneous space within the interface.

For any of the methods described herein, providing the *in vitro* mammalian skin may comprise immobilizing the *in vitro* mammalian skin between a proximal substrate and a distal substrate. In some methods, the proximal substrate and the distal substrate each comprise glass, such as glass cover slips.

For any of the methods described herein, providing the *in vitro* mammalian skin may comprise providing the dermal layer of the skin model disposed on a porous substrate that comprises pores sized to accommodate diffusion of the therapeutic compound product. The administering may comprise placing the porous substrate in fluid communication with a solution comprising the therapeutic compound product. The method may further comprise detecting a presence of level of subvisible particles in the solution after said administering.

For any of the methods described herein, the *in vitro* mammalian skin may be an *in vitro* human skin. For any of the methods described herein, the *in vitro* mammalian skin may be an *in vitro* skin of a non-human primate, such as a cynomolgus monkey. For any of the methods described herein, the *in vitro* mammalian skin may be of a non-human mammal. For some methods in which the *in vitro* mammalian skin is of a non-human mammal, the method may further comprise repeating the method using an *in vitro* human skin, and comparing physiochemical and/or clinical characteristics of the therapeutic compound administered to the *in vitro* human skin with those of the therapeutic compound administered to the *in vitro* mammalian skin is of the non-human mammal.

For any of the methods described herein, the detecting may comprise imaging and/or analytical testing. The analytical testing may comprise mass spectrometry, chromatography, electrophoresis, spectroscopy, light obscuration, a particle method (such as nanoparticle/visible/micron-sized resonant mass or Brownian motion), analytical centrifugation, imaging or imaging characterization, immunoassay, SE-HPLC, rCE-SDS, CEX-HPLC, HIAC, nrCE-SDS, mass spectrometry microscopy, and/or mass spectroscopy. The imaging may comprise CT scan or magnetic resonance imaging. For any of the methods described herein, the detecting may comprise optical imaging. For any of the methods described herein, the detecting may comprise obtaining a background signal and subtracting the background signal.

For any of the methods described herein, the method may further comprise labeling the therapeutic compound with a detectable moiety such as a fluorophore, fluorescent dye, radiolabel, or quantum dot.

According to the invention, the therapeutic compound comprises an antibody, an antigen-binding antibody fragment, an antibody protein product, a Bi-specific T cell engager (BiTE^{®}) molecule or a bispecific antibody.

For any of the methods described herein, the method may further comprise performing analytical testing on the therapeutic compound prior to administering the therapeutic compound to the interface.

### BRIEF DESCRIPTION OF THE DRAWINGS

**FIGs. 1A-B** is a series of schematic diagrams illustrating an injection and fluorescence-based approach to resolve therapeutic compound behavior in the *in vitro* mammalian skin environment of some embodiments. **FIG. 1A** depicts fluorescent labeling of the therapeutic compounds, which is suitable for some methods described herein. It will also be appreciated that some methods described herein may be performed on unlabeled therapeutic compounds. **FIG. 1B** illustrates options for imaging in addition to those shown in **FIG. 1A****.**
**FIGs. 1C-1E** are flow diagrams illustrating methods of some embodiments.
**FIGs. 2A-D** are a series of image overlays of mAbs with varying pH-sensitive aggregation propensities injected into *in vitro* mammalian skin according to some embodiments. Arrowheads show aggregates of mAbs.
**FIGs. 3A-B** are a series of schematic diagrams illustrating exposure and analytical strategies to resolve attribute changes following *in vitro* mammalian skin exposure according to some embodiments.
**FIGs. 4A-D** are a series of graphs showing comparisons of high molecular weight (HMW) species and subvisible particle (SbVP) levels for two formulations of mAb3 exposed to *in vitro* mammalian skin of some embodiments.
**FIGs. 5A-C** are a series of graphs showing comparisons of high molecular weight (HMW) species and subvisible particle (SbVP) levels for several therapeutic proteins with varying formulations.
**FIG. 6** is a series of images of therapeutic protein solutions injected into *in vitro* mammalian skin according to some embodiments.

### DETAILED DESCRIPTION

Described herein are methods of analyzing, selecting, and characterizing therapeutic compounds and pharmaceutical compositions comprising the same. Prior to the present application, assessment of therapeutic compound pharmaceutical composition interactions within the SubQ space have been limited by the lack of robust experimental systems that adequately capture the biological complexity of human skin. Accordingly, prior to the present application, there has been a need for accurate methods of assessing attributes of therapeutic compounds, such as aggregation and solubility in a SubQ environment. Described herein are methods that use *in vitro* mammalian skin such as human skin for modeling the impact of the skin environment on therapeutic compounds (and pharmaceutical compositions comprising the same) in the SubQ space. The methods can be used for selecting a condition of administration for therapeutic compound product (such as one or more formulation characteristics, or a device or route of administration), determining an impact of a molecular attribute (such as HMW species) of a therapeutic compound on a physiochemical and/or clinical characteristic of the therapeutic compound after subcutaneous administration, and/or detecting a change in a molecular attribute of a therapeutic compound after subcutaneous administration. The methods can comprise providing a live *in vitro* mammalian skin comprising a dermal layer and epidermal layer that define an interface between the dermal and epidermal layer. The methods can comprise administering a therapeutic compound product to the interface. The methods can comprise detecting a physiochemical and/or clinical characteristic of the therapeutic compound product in the interface after it has been administered. The therapeutic compound product may acquire the physiochemical and/or clinical characteristic after being administered. Examples of physiochemical and/or clinical characteristics include bioavailability, pharmacokinetic property, aggregation, chemical modification, precipitation, distribution, diffusion rate, retention and/or absorption. Advantageously, the methods described herein can robustly determine the impact of a subcutaneous environment on therapeutic compounds and pharmaceutical compositions comprising therapeutic compounds with fidelity and consistency. For example, it is contemplated that the methods describe herein can model a subcutaneous environment with greater fidelity than *in vitro* buffered solutions alone, and with greater consistency and reproducibility than live animal models.

Protein aggregation and poor solubility can be major hurdles for drug manufacturing and delivery. Therapeutically effective doses often require high protein concentrations that are achieved through extensively developed formulations that vary significantly from physiological conditions. These formulations commonly include acidic pH (≈5.0) and stabilizing excipients that have been optimized to maintain the necessary solubility, stability, and viscosity profiles for the biologic solutions to be delivered to patients. Upon injection into skin, a variety of factors in the local environment may affect how biologics become distributed within the body. Once the drug depot is formed following injection, the initial changes in the solubility and stability of the biologics may be driven by the loss of buffering components and stabilizing excipients that diffuse away from the injection site at faster rates than the biologics. Contact with salts, proteins, cells, and extracellular constituents of the skin environment may provide new interactions that induce aggregation and impact the bulk solution behavior of the drug. *In vitro* skin models provide a useful approach for predicting issues related to drug distributions within skin and present opportunities to screen for conditions that may affect stability, clinical pharmacokinetics, and bioavailability.

*In vitro* studies are serving increasingly larger roles in therapeutic compound development by providing efficient and cost-effective means to optimize pharmaceutical composition performance and identify potential issues that may affect product quality and efficacy. With the establishment of subcutaneous injection as a prominent administration route for biologics, there is a growing need to identify and better understand drug interactions within the skin environment.^{[1]} The use of tissue engineering permits the production of *in vitro* mammalian skin that recapitulates many of the morphological and functional features of human skin.^{[2]} The in *vitro* mammalian skin can be produced using open source protocols^{[2]} or purchased from commercial vendors.^{[3][4]} Lab-grown skin models generally include separate models for each of the three distinct layers of human skin (epidermis^{[5]}, dermis^{[6]}, and hypodermis^{[7]}), or models that comprise the epidermis and dermis (but lack the hypodermis)^{[8]}. Furthermore, development of models containing all three layers has been described^{[9]}. In the cosmetology and skin care industries, *in vitro* skin models focusing on the outer layers have been used for toxicological and formulation studies^{[10][11]}, however, these models have generally not been used to examine the impact of skin environments on injectable therapeutic compounds.

The methods described herein use *in vitro* mammalian skin such as human skin to provide insight on the impact of the skin environment on the physicochemical properties of therapeutic compounds and pharmaceutical compositions comprising therapeutic compounds. Some methods described herein involve the use of imaging such as fluorescence imaging to characterize the physiochemical and/or clinical characteristics of therapeutic compounds such as aggregation propensities and diffusion properties upon injection into skin. These *in vitro* approaches permit the detection of potential aggregation issues and biodistribution challenges within skin-like environments that may affect drug absorption. Some methods described herein involve the use of analytical measurements (e.g., chromatography-based assays, particle counting, and/or mass spectrometry) to characterize changes in molecular attributes after subcutaneous administration, and/or the impact of molecular attributes on physiochemical and/or clinical characteristic of the therapeutic compound after subcutaneous administration to *in vitro* mammalian skin. The *in vitro* mammalian skin in these applications may be used, for example, to optimize therapeutic compounds and pharmaceutical compositions comprising therapeutic compounds, screen formulations, and improve injection strategies for subcutaneously administered biotherapeutics.

### In vitro Mammalian Skin

*In vitro* mammalian skin may be used in methods described herein. As used herein *"in vitro* mammalian skin" refers to live mammalian skin comprising a dermal layer and epidermal layer that define an interface between the dermal and epidermal layer. It may be provided in an *in vitro* environment, for example disposed in a multi-well plate. The *in vitro* mammalian skin may be disposed on a substrate such as a glass or polymer cover slip, or a porous substrate. Alternatively, the *in vitro* mammalian skin may be suspended in media in the absence of a substrate. In some methods, the *in vitro* mammalian skin comprises three layers epidermis^{[5]}, dermis^{[6]}, and hypodermis^{[7]}. These three layers may be provided together, or may be provided as separate layers. In some methods, the *in vitro* mammalian skin comprises epidermis and dermis, but lacks hypodermis.^{[8]}

For any of the methods described herein, the *in vitro* mammalian skin may further comprise a hypodermis that defines a subcutaneous space within the interface. It will be appreciated that the subcutaneous space refers to a portion of the interface that is further defined, at least in part, by a hypodermis. As such, in the methods of some embodiments, the interface of the *in vitro* mammalian skin further comprises a subcutaneous space, which may define a subcutaneous space. It is also contemplated that *in vitro* mammalian skin comprising a dermal layer and epidermal layer may provide useful modeling relevant to a subcutaneous environment, even if the hypodermis is not present.

By way of example, the *in vitro* mammalian skin model may be the skin of a human, non-human primate (such as cynomolgus monkey), or a non-human mammal (such as mouse, rat, rabbit, dog, goat, sheep, or pig). In some methods, the *in vitro* mammalian skin may be the skin of a human, or of a non-human primate such as cynomolgus monkey. In the development of therapeutic compounds, the therapeutic compounds may be examined in animal models (e.g., non-human primates such as cynomolgus monkeys), for example in toxicology and/or efficacy studies. Accordingly, it is contemplated that the *in vitro* mammalian skin describe herein may be useful in selection and development of therapeutic compounds and pharmaceutical compositions comprising therapeutic compounds prior to animal model studies, for example *in vitro* aggregation, pharmacokinetic and/or toxicology studies using an *in vitro* mammalian skin of the same species as the animal model.

For any of the methods described herein, the *in vitro* mammalian skin model may be the skin of a non-human mammal. The method may further be repeated using the *in vitro* human skin. The method may comprise comparing physiochemical and/or clinical characteristics of the therapeutic compound administered to the *in vitro* human skin with those of the therapeutic compound administered to the *in vitro* mammalian skin of a non-human mammal. That is, the method may compare the behavior of the therapeutic compound in the *in vitro* mammalian skin of the non-human mammal to that of the *in vitro* human skin to ascertain the suitability of the non-human mammal for further experiments. For any of the methods described herein, the *in vitro* mammalian skin may comprise or consist of *in vitro* human skin. For any of the methods described herein, the *in vitro* mammalian skin may comprise or consist of an *in vitro* skin of a non-human primate, such as a cynomolgus monkey.

### Therapeutic Compounds

As used herein "therapeutic compound" and variations of this root term has its ordinary and customary meaning as would be understood by one of ordinary skill in the art in view of this disclosure. It refers to a therapeutic composition comprising an active pharmaceutical ingredient, for example a synthetic small molecule, a gene therapy, a therapeutic protein, a nucleic acid such as antisense RNA or siRNA, a virus, or a cell or a portion thereof.

As used herein "therapeutic protein" and variations of this root term has its ordinary and customary meaning as would be understood by one of ordinary skill in the art in view of this disclosure. It refers to a therapeutic composition comprising a polypeptide macromolecule, for example a protein, peptide, or portion thereof. A therapeutic protein may be a protein for medical use in a human subject, such as a candidate for medical use, or a protein approved for medical use by a government authority such as the FDA or EMA. It will be appreciated that a therapeutic protein is a type of therapeutic compound. Accordingly, wherever therapeutic compounds are mentioned herein, therapeutic proteins such as those described here are contemplated.

In methods described herein, the therapeutic compound may comprise or consist of a therapeutic protein. According to the invention, the therapeutic compound may be selected from the group consisting of: an antibody, an antigen-binding antibody fragment, an antibody protein product, a Bi-specific T cell engager (BiTE^{®}) molecule (such as a BiTE^{®} molecule comprising a half-life extension moiety), and a bispecific antibody.

An "antibody" has its customary and ordinary meaning as understood by one of ordinary skill in the art in view of this disclosure. It refers to an immunoglobulin of any isotype with specific binding to the target antigen, and includes, for instance, chimeric, humanized, and fully human antibodies. By way of example, the antibody may be a monoclonal antibody. The human antibodies can be of any isotype, including IgG (including IgG1, IgG2, IgG3 and IgG4 subtypes), IgA (including IgA1 and IgA2 subtypes), IgM and IgE. A human IgG antibody generally will comprise two full-length heavy chains and two full-length light chains. Antibodies may be derived solely from a single source, or may be "chimeric," that is, different portions of the antibody may be derived from two or more different antibodies from the same or different species. It will be understood that once an antibody is obtained from a source, it may undergo further engineering, for example to enhance stability and folding. Accordingly, it will be understood that a "human" antibody may be obtained from a source, and may undergo further engineering, for example in the Fc region. The engineered antibody may still be referred to as a type of human antibody. Similarly, variants of a human antibody, for example those that have undergone affinity maturation, will also be understood to be "human antibodies" unless stated otherwise. In some embodiments, an antibody comprises, consists essentially of, or consists of a human, humanized, or chimeric monoclonal antibody.

A "heavy chain" of an antibody (or an antibody protein product) includes a variable region ("VH"), and three constant regions: CH1, CH2, and CH3. Example heavy chain constant regions include human IgG1, IgG2, IgG3, and IgG4 constant regions. A "light chain" of an antibody (or an antibody protein product) includes a variable region ("VL"), and a constant region ("CL"). Human light chains include kappa chains and lambda chains. Example light chain constant regions suitable for antigen binding proteins described herein include human lambda and human kappa constant regions.

In various aspects, the therapeutic protein is an antibody protein product. As used herein, the term "antibody protein product" refers to any one of several antibody alternatives which in various instances is based on the architecture of an antibody but is not found in nature. In some aspects, the antibody protein product has a molecular-weight within the range of at least about 12-150 kDa. In certain aspects, the antibody protein product has a valency (n) range from monomeric (n = 1), to dimeric (n = 2), to trimeric (n = 3), to tetrameric (n = 4), if not higher order valency. Antibody protein products in some aspects are those based on the full antibody structure and/or those that mimic antibody fragments which retain full antigen-binding capacity, e.g., scFvs, Fabs and VHH/VH (discussed below). The smallest antigen binding antibody fragment that retains its complete antigen binding site is the Fv fragment, which consists entirely of variable (V) regions. A soluble, flexible amino acid peptide linker is used to connect the V regions to a scFv (single chain fragment variable) fragment for stabilization of the molecule, or the constant (C) domains are added to the V regions to generate a Fab fragment [fragment, antigen-binding]. Both scFv and Fab fragments can be easily produced in host cells, e.g., prokaryotic host cells. Other antibody protein products include disulfide-bond stabilized scFv (ds-scFv), single chain Fab (scFab), as well as di- and multimeric antibody formats like dia-, tria-and tetra-bodies, or minibodies (miniAbs) that comprise different formats consisting of scFvs linked to oligomerization domains. The smallest fragments are VHH/VH of camelid heavy chain Abs as well as single domain Abs (sdAb). The building block that is most frequently used to create novel antibody formats is the single-chain variable (V)-domain antibody fragment (scFv), which comprises V domains from the heavy and light chain (VH and VL domain) linked by a peptide linker of ^{~}15 amino acid residues. A peptibody or peptide-Fc fusion is yet another antibody protein product. The structure of a peptibody consists of a biologically active peptide grafted onto an Fc domain. Peptibodies are well-described in the art. See, e.g., Shimamoto et al., mAbs 4(5): 586-591 (2012).

Therapeutic proteins suitable for the methods described herein can include polypeptides, including those that bind to one or more of the following: These include CD proteins, including CD3, CD4, CD8, CD19, CD20, CD22, CD30, and CD34; including those that interfere with receptor binding. HER receptor family proteins, including HER2, HER3, HER4, and the EGF receptor. Cell adhesion molecules, for example, LFA-1, Mol, pl50, 95, VLA-4, ICAM-I, VCAM, and alpha v/beta 3 integrin. Growth factors, such as vascular endothelial growth factor ("VEGF"), growth hormone, thyroid stimulating hormone, follicle stimulating hormone, luteinizing hormone, growth hormone releasing factor, parathyroid hormone, Mullerian-inhibiting substance, human macrophage inflammatory protein (MIP-1alpha), erythropoietin (EPO), nerve growth factor, such as NGF-beta, platelet-derived growth factor (PDGF), fibroblast growth factors, including, for instance, aFGF and bFGF, epidermal growth factor (EGF), transforming growth factors (TGF), including, among others, TGF-α and TGF-β, including TGF-βI, TGF-β2, TGF-β3, TGF-β4, or TGF-β5, insulin-like growth factors-I and -II (IGF-I and IGF-II), des(I-3)-IGF-I (brain IGF-I), and osteoinductive factors. Insulins and insulin-related proteins, including insulin, insulin A-chain, insulin B-chain, proinsulin, and insulin-like growth factor binding proteins. Coagulation and coagulation-related proteins, such as, among others, factor VIII, tissue factor, von Willebrands factor, protein C, alpha-1-antitrypsin, plasminogen activators, such as urokinase and tissue plasminogen activator ("t-PA"), bombazine, thrombin, and thrombopoietin; (vii) other blood and serum proteins, including but not limited to albumin, IgE, and blood group antigens. Colony stimulating factors and receptors thereof, including the following, among others, M-CSF, GM-CSF, and G-CSF, and receptors thereof, such as CSF-1 receptor (c-fms). Receptors and receptor-associated proteins, including, for example, flk2/flt3 receptor, obesity (OB) receptor, LDL receptor, growth hormone receptors, thrombopoietin receptors ("TPO-R," "c-mpl"), glucagon receptors, interleukin receptors, interferon receptors, T-cell receptors, stem cell factor receptors, such as c-Kit, and other receptors. Receptor ligands, including, for example, OX40L, the ligand for the OX40 receptor. Neurotrophic factors, including bone-derived neurotrophic factor (BDNF) and neurotrophin-3, -4, -5, or -6 (NT-3, NT-4, NT-5, or NT-6). Relaxin A-chain, relaxin B-chain, and prorelaxin; interferons and interferon receptors, including for example, interferon-α, -β, and -γ, and their receptors. Interleukins and interleukin receptors, including IL-1 to IL-33 and IL-1 to IL-33 receptors, such as the IL-8 receptor, among others. Viral antigens, including an AIDS envelope viral antigen. Lipoproteins, calcitonin, glucagon, atrial natriuretic factor, lung surfactant, tumor necrosis factor-alpha and -beta, enkephalinase, RANTES (regulated on activation normally T-cell expressed and secreted), mouse gonadotropin-associated peptide, DNAse, inhibin, and activin. Integrin, protein A or D, rheumatoid factors, immunotoxins, bone morphogenetic protein (BMP), superoxide dismutase, surface membrane proteins, decay accelerating factor (DAF), HIV envelope, transport proteins, homing receptors, addressins, regulatory proteins, immunoadhesins, antibodies. Myostatins, TALL proteins, including TALL-I, amyloid proteins, including but not limited to amyloid-beta proteins, thymic stromal lymphopoietins ("TSLP"), RANK ligand ("RANKL" or "OPGL"), c-kit, TNF receptors, including TNF Receptor Type 1, TRAIL-R2, angiopoietins, and biologically active fragments or analogs or variants of any of the foregoing.

Examples of therapeutic proteins suitable for the methods described herein include antibodies such as abagovomab, abciximab, actoxumab, adalimumab, afelimomab, afutuzumab, alacizumab, alacizumab pegol, ald518, alemtuzumab, alirocumab, altumomab, amatuximab, anatumomab mafenatox, anrukinzumab, apolizumab, arcitumomab, aselizumab, altinumab, atlizumab, atorolimiumab, tocilizumab, bapineuzumab, basiliximab, bavituximab, bectumomab, belimumab, bemarituzumab, benralizumab, bertilimumab, besilesomab, bevacizumab, bezlotoxumab, biciromab, bivatuzumab, bivatuzumab mertansine, blinatumomab, blosozumab, brentuximab vedotin, briakinumab, brodalumab, canakinumab, cantuzumab mertansine, cantuzumab mertansine, caplacizumab, capromab pendetide, carlumab, catumaxomab, cc49, cedelizumab, certolizumab pegol, cetuximab, citatuzumab bogatox, cixutumumab, clazakizumab, clenoliximab, clivatuzumab tetraxetan, conatumumab, crenezumab, cr6261, dacetuzumab, daclizumab, dalotuzumab, daratumumab, demcizumab, denosumab, detumomab, dorlimomab aritox, drozitumab, duligotumab, dupilumab, ecromeximab, eculizumab, edobacomab, edrecolomab, efalizumab, efungumab, elotuzumab, elsilimomab, enavatuzumab, enlimomab pegol, enokizumab, enoticumab, ensituximab, epitumomab cituxetan, epratuzumab, erenumab, erlizumab, ertumaxomab, etaracizumab, etrolizumab, evolocumab, exbivirumab, fanolesomab, faralimomab, farletuzumab, fasinumab, fbta05, felvizumab, fezakinumab, ficlatuzumab, figitumumab, flanvotumab, fontolizumab, foralumab, foravirumab, fresolimumab, fulranumab, futuximab, galiximab, ganitumab, gantenerumab, gavilimomab, gemtuzumab ozogamicin, gevokizumab, girentuximab, glembatumumab vedotin, golimumab, gomiliximab, gs6624, ibalizumab, ibritumomab tiuxetan, icrucumab, igovomab, imciromab, imgatuzumab, inclacumab, indatuximab ravtansine, infliximab, intetumumab, inolimomab, inotuzumab ozogamicin, ipilimumab, iratumumab, itolizumab, ixekizumab, keliximab, labetuzumab, lebrikizumab, lemalesomab, lerdelimumab, lexatumumab, libivirumab, ligelizumab, lintuzumab, lirilumab, lorvotuzumab mertansine, lucatumumab, lumiliximab, mapatumumab, maslimomab, mavrilimumab, matuzumab, mepolizumab, metelimumab, milatuzumab, minretumomab, mitumomab, mogamulizumab, morolimumab, motavizumab, moxetumomab pasudotox, muromonab-cd3, nacolomab tafenatox, namilumab, naptumomab estafenatox, narnatumab, natalizumab, nebacumab, necitumumab, nerelimomab, nesvacumab, nimotuzumab, nivolumab, nofetumomab merpentan, ocaratuzumab, ocrelizumab, odulimomab, ofatumumab, olaratumab, olokizumab, omalizumab, onartuzumab, oportuzumab monatox, oregovomab, orticumab, otelixizumab, oxelumab, ozanezumab, ozoralizumab, pagibaximab, palivizumab, panitumumab, panobacumab, parsatuzumab, pascolizumab, pateclizumab, patritumab, pemtumomab, perakizumab, pertuzumab, pexelizumab, pidilizumab, pintumomab, placulumab, ponezumab, priliximab, pritumumab, PRO 140, quilizumab, racotumomab, radretumab, rafivirumab, ramucirumab, ranibizumab, raxibacumab, regavirumab, reslizumab, rilotumumab, rituximab, robatumumab, roledumab, romosozumab, rontalizumab, rovelizumab, ruplizumab, samalizumab, sarilumab, satumomab pendetide, secukinumab, sevirumab, sibrotuzumab, sifalimumab, siltuximab, simtuzumab, siplizumab, sirukumab, solanezumab, solitomab, sonepcizumab, sontuzumab, stamulumab, sulesomab, suvizumab, tabalumab, tacatuzumab tetraxetan, tadocizumab, talizumab, tanezumab, taplitumomab paptox, tefibazumab, telimomab aritox, tenatumomab, tefibazumab, teneliximab, teplizumab, teprotumumab, tezepelumab, TGN1412, tremelimumab, ticilimumab, tildrakizumab, tigatuzumab, TNX-650, tocilizumab, toralizumab, tositumomab, tralokinumab, trastuzumab, TRBS07, tregalizumab, tucotuzumab celmoleukin, tuvirumab, ublituximab, urelumab, urtoxazumab, ustekinumab, vapaliximab, vatelizumab, vedolizumab, veltuzumab, vepalimomab, vesencumab, visilizumab, volociximab, vorsetuzumab mafodotin, votumumab, zalutumumab, zanolimumab, zatuximab, ziralimumab, or zolimomab aritox.

In some embodiments, the therapeutic protein is a BiTE^{®} molecule. BiTE^{®} molecules are engineered bispecific antigen binding constructs which direct the cytotoxic activity of T cells against cancer cells. They contain the fusion of two single-chain variable fragments (scFvs) of different antibodies, or amino acid sequences from four different genes, on a single peptide chain of about 55 kilodaltons. One of the scFvs binds to T cells via the CD3 receptor, and the other to a tumor cell via a tumor specific molecule. Blinatumomab (BLINCYTO^{®}) is an example of a BiTE^{®} molecule, specific for CD19. BiTE^{®} molecules that are modified, such as those modified to extend their half-lives (e.g., by comprising a half-life extension moiety such as an Fc polypeptide or albumin), can also be used in the disclosed methods. In various aspects, the polypeptide is an antigen binding protein, e.g., a BiTE^{®} molecule. In some embodiments, an antibody protein product comprises a BiTE^{®} molecule.

Additional examples of therapeutic compounds include synthetic small molecules such as carfilzomib, cinacalcet HCl, etelcalcetide, ivabradine, sotorasib, imatinib mesylate, bortezomib, bicalutamide, gefitinib, venetoclax, and docorubicin; and siRNAs such as olpasiran.

It will be appreciated that therapeutic compounds are typically administered in patients in pharmaceutical compositions, which may also be referred to as formulations. Accordingly, methods herein may be used to study not only impact of the skin environment on the therapeutic compound itself, but also on the pharmaceutical composition comprising the therapeutic compound. Accordingly, wherever a "therapeutic compound" is described herein, it will be appreciated that a method described herein may also be implemented on a corresponding pharmaceutical composition comprising the therapeutic compound. For example, the methods described herein may be used to examine the impact of the skin environment on different therapeutic compound formulation ingredients, amounts, and conditions (such as pH, viscosity, osmolarity, osmolality, and the like) for the development and selection of suitable formulations.

In methods of some embodiments, the therapeutic compound is in a pharmaceutical composition, which may also be referred to as a "formulation." The pharmaceutical composition may be a pharmaceutically acceptable formulation. The pharmaceutical composition may comprise the therapeutic compound together with a pharmaceutically acceptable diluent, carrier, solubilizer, emulsifier, preservative, and/or adjuvant.

Acceptable pharmaceutical composition materials for therapeutic compounds as described herein preferably are nontoxic to recipients at the dosages and concentrations employed. In certain embodiments, the pharmaceutical composition may contain formulation materials for modifying, maintaining or preserving, for example, the pH, osmolality, viscosity, clarity, color, isotonicity, odor, sterility, stability, rate of dissolution or release, adsorption or penetration of the composition. In such embodiments, suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, sucrose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. *See, e.g.,* REMINGTON'S PHARMACEUTICAL SCIENCES, 18th Edition, (A. R. Gennaro, ed.), 1990, Mack Publishing Company.

A suitable vehicle or carrier for the pharmaceutical composition may be water for injection, physiological saline solution or artificial cerebrospinal fluid, possibly supplemented with other materials common in compositions for parenteral administration. Neutral buffered saline or saline mixed with serum albumin are further exemplary vehicles. In specific embodiments, pharmaceutical compositions comprise Tris buffer of about pH 7.0-8.5, or acetate buffer of about pH 4.0-5.5, and may further include sorbitol or a suitable substitute therefor.

The pharmaceutical composition components are present preferably in concentrations that are acceptable to the site of administration. In certain embodiments, buffers are used to maintain the composition at physiological pH or at a slightly lower pH, typically within a pH range of from about 4 to about 8. Including about 4.1, about 4.2, about 4.3, about 4.4, about 4.5, about 4.6, about 4.7, about 4.8, about 4.9, about 5.0, 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, and about 8.0, including ranges between any two of the listed values, for example pH 4.2 to 8, pH 4.2 to 7, pH 4.2 to 6, pH 4.2 to 5, pH 4.8 to 8, pH 4.8 to 7, pH 4.8 to 6, pH 4.8 to 5, pH 5 to 8, pH 5 to 7, pH 5 to 6, pH 5.1 to 8, pH 5.1 to 7, pH 5.1 to 6, pH 5.5 to 8, pH 5.5 to 7, pH 5.5 to 6, pH 6 to 7, or pH 6 to 8.

It is noted that some therapeutic compounds may be self-administered by subjects themselves either directly or via an automated injector (such as an on-body injector), while some may be administered to the subject by another individual such as a health care provider. As such, a subject therapeutic compound or pharmaceutical composition comprising the same may be suitable for self-administration by the subjects to themselves (either directly, or via an apparatus such as an automated injector), and/or by other individuals, such as health care providers, to the subject. For some methods described herein, pharmaceutical composition comprising the therapeutic compound may be provided in an administration apparatus such as a pre-filled syringe, autoinjector, or IV bag system comprising a needle in fluid communication with an IV bag.

### Molecular attributes

It will be appreciated that a "molecular attribute" of a therapeutic compound such as a therapeutic protein refers to one or more chemical or structural characteristic of the therapeutic protein, which may vary. Examples of molecular attributes include acidic species, basic species, high molecular weight species, subvisible particle number, low molecular weight, middle molecular weight, glycosylation (such as non-glycosylated heavy chain or high mannose), non-heavy chain and light chain, deamidation, deamination, cyclization, oxidation, isomerization, fragmentation/clipping, N-terminal and C-terminal variants, reduced and partial species, folded structure, surface hydrophobicity, chemical modification, covalent bond, a C-terminal amino acid motif PARG, and/or C-terminal amino acid motif PAR-Amide. By way of example, it is noted that therapeutic proteins and small molecules have been observed to aggregate, and nucleic acids have been observed in multiplex, which for conciseness may be considered a type of "aggregate."

### Methods of selecting conditions of administration

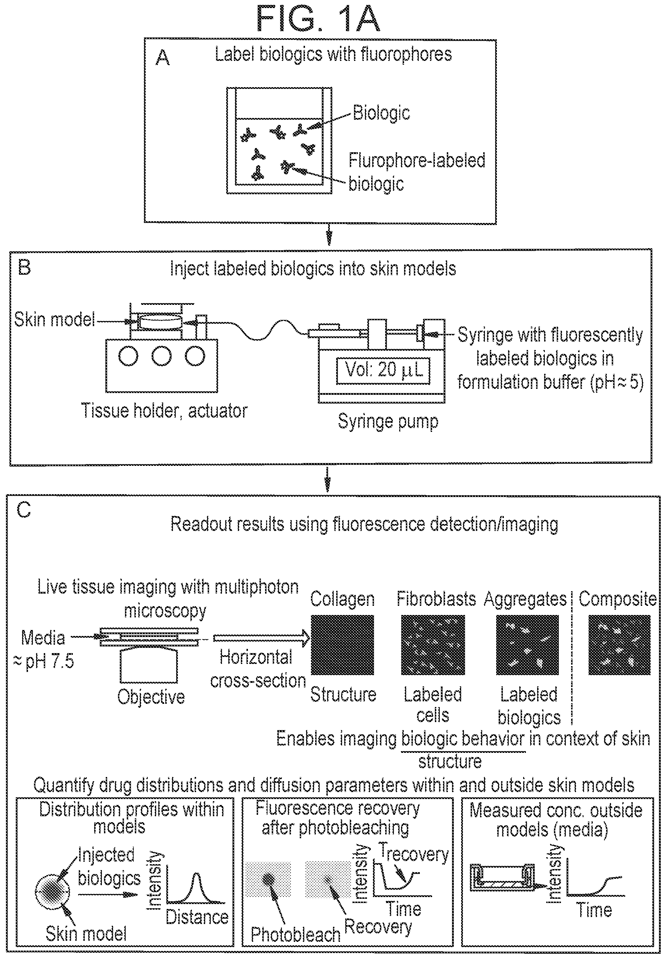

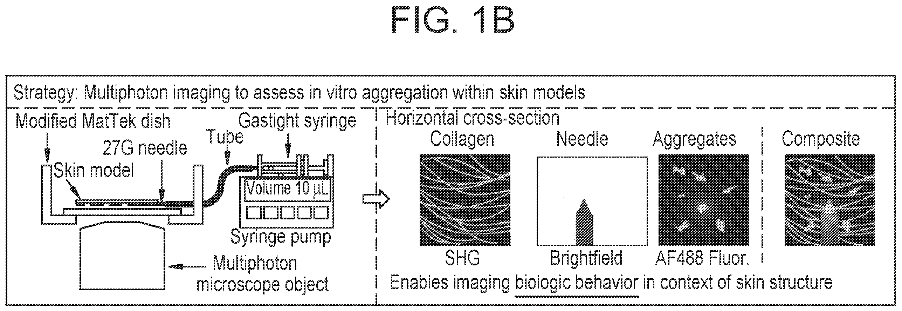

In some embodiments, methods of selecting a condition of administration for a therapeutic compound product for subcutaneous administration are described. As used herein, "a condition of administration" refers to at least one parameter that can vary when a therapeutic compound (e.g., as part of a pharmaceutical composition) is administered. Examples of conditions of administration include one or more of formulation ingredient, concentration, volume, pH, temperature, viscosity, osmolarity, a molecular attribute of said therapeutic compound, route of administration, and/or administration apparatus or method of using an administration apparatus (e.g. frequency, flow rate, parameter). For example, the condition of administration may include an excipient, buffer, surfactant, and/or stabilizer of a formulation, which may vary by identity and/or concentration of these substances, and/or may also include pH It will be appreciated that as applicable, a condition of administration may be identified qualitatively (e.g., a route of administration) or quantitatively (for example, as a numerical value or range). For illustrative purposes, examples of methods of resolving therapeutic compound behavior in an *in vitro* mammalian skin environment of some embodiments are illustrated in **FIGs. 1A-B****.**

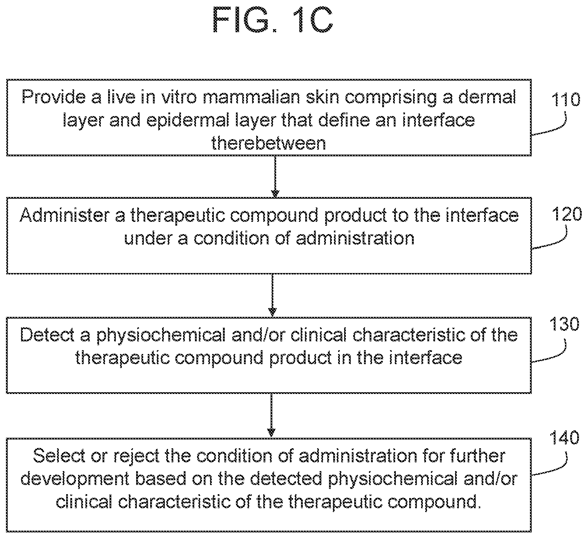

Example methods of selecting a condition of administration for a therapeutic compound product for subcutaneous administration in conjunction with some embodiments are illustrated in **FIG. 1C****.** The method may comprise providing a live *in vitro* mammalian skin comprising a dermal layer and epidermal layer that define an interface therebetween (that is, between the dermal and epidermal layer) **110.** The method may further comprise administering a therapeutic compound product to the interface under a condition of administration **120.** The method may further comprise detecting a physiochemical and/or clinical characteristic of the therapeutic compound product in the interface **130.** For example, the detecting may be optical, and/or may comprise analytical testing of the therapeutic compound. The method may further comprise selecting or rejecting the condition of administration for further development based on the detected physiochemical and/or clinical characteristic of the therapeutic compound **140.** For example, a condition of administration that has a lower incidence of HMW species may be selected over that which has a higher incidence of HMW species. "Further development," as used herein refers to additional characterization, engineering, and/or study of the therapeutic compound, for example, protein engineering, formulation selection and/or development, animal model study, clinical study, manufacturing process development, and/or regulatory submission. It will be understood that as appropriate in context, one or more of the noted portions of the method may be repeated or omitted or performed in a difference sequence.

It will be appreciated that the method of selecting a condition of administration may detect one or more physiochemical and/or clinical characteristic of the therapeutic compound, including changes in the physiochemical and/or clinical characteristic. For example, the physiochemical and/or clinical characteristic may comprise bioavailability, pharmacokinetic property, aggregation, precipitation, distribution, diffusion rate, retention and/or absorption. By way of example, the method may detect an absolute amount, relative amount, change therein, or rate of change of one or more physiochemical and/or clinical characteristic of the therapeutic compound, as applicable, and as makes sense in context. The therapeutic compound product may acquire the physiochemical and/or clinical characteristic after being administered.

In some methods of selecting a condition of administration for a therapeutic compound product for subcutaneous administration, the method is repeated for two or more different conditions of administration, for example at least two, three, four, five, six, seven, eight, nine, or ten different conditions of administration. Optionally, the method may be performed iteratively to optimize a condition of administration (for example iteratively testing narrower and narrower ranges of a condition of administration).

### Methods of determining an impact of a molecular attribute of a therapeutic compound on a physiochemical and/or clinical characteristic

In some embodiments, methods of determining an impact of a molecular attribute of a therapeutic compound on a physiochemical and/or clinical characteristic of the therapeutic compound after subcutaneous administration are described.

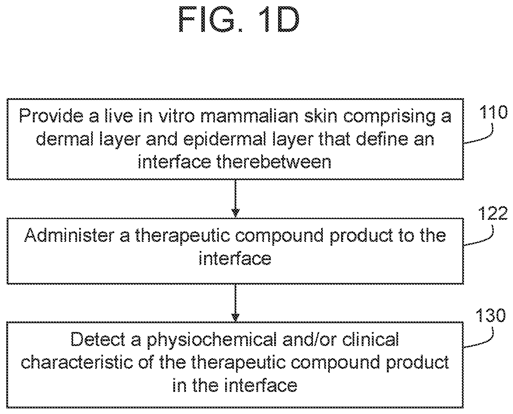

Example methods of determining an impact of a molecular attribute of a therapeutic compound on a physiochemical and/or clinical characteristic of a therapeutic compound after subcutaneous administration in conjunction with some embodiments are illustrated in **FIG. 1D****.** The method may comprise providing a live *in vitro* mammalian skin comprising a dermal layer and epidermal layer that define an interface therebetween (that is, between the dermal and epidermal layer) **110.** The method may further comprise administering a therapeutic compound product comprising a molecular attribute to the interface **122.** It is contemplated that the therapeutic compound being administered may be stressed, for example by exposure to elevated temperature, repeated freeze-thaw cycles, extremes of pH, forced oxidation (e.g., with an oxidizing agent such as H₂O₂), physiological pH, and/or UV light. The stress may cause the molecular attribute to be present on the therapeutic compound, or may increase the relative abundance of the molecular attribute compared to an unstressed control. Accordingly, in some instances of the method, the therapeutic compound may be stressed prior to administration. The method may comprise detecting a physiochemical and/or clinical characteristic of the therapeutic compound in the interface **130.** For example, the detecting may be optical, and/or may comprise analytical testing of the therapeutic compound. In some methods of determining an impact of a molecular attribute of a therapeutic compound on a physiochemical and/or clinical characteristic of the therapeutic compound, the physiochemical and/or clinical characteristic comprises bioavailability, pharmacokinetic property, aggregation, precipitation, distribution, diffusion rate, and/or absorption. It will be understood that as appropriate in context, one or more of the noted portions of the method may be repeated or omitted or performed in a difference sequence.

### Methods of detecting a change in a molecular attribute

In some embodiments, methods of detecting a change in a molecular attribute of a therapeutic compound after subcutaneous administration are described.

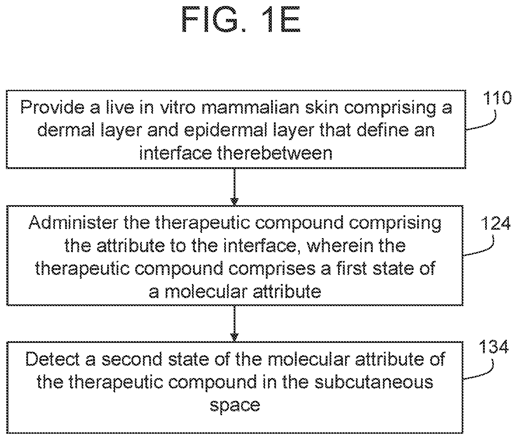

Example methods of detecting a change in a molecular attribute of a therapeutic compound after subcutaneous administration in conjunction with some embodiments are illustrated in **FIG. 1E****.** The method may comprise providing a live *in vitro* mammalian skin comprising a dermal layer and epidermal layer that define an interface therebetween (that is, between the dermal and epidermal layer) **101.** The method may comprise administering the therapeutic compound comprising the attribute to the interface, in which the therapeutic compound comprises a first state of a molecular attribute **124.** Turning to block **134,** the method may further comprise detecting a second state of the molecular attribute of the therapeutic compound in the interface **134.** It will be understood that as appropriate in context, one or more of the noted portions of the method may be repeated or omitted or performed in a difference sequence. The "first state" and "second state" refer to two states of a molecular attribute that may be measured. For example, the first state and second state of the molecular attribute comprise any of concentration, quantity, distribution on the therapeutic compound, or distribution in the interface. For example, the first state may refer to a first measurement of relative quantity of high molecular weight (HMW) species prior to administration to the interface (or immediately upon administration to the interface), and the second state may refer to a second measurement of relative quantity of high molecular weight (HMW) species after administration to the interface.

The value (quantitative and/or qualitative) of the first state may differ from that of the second state, or the values may be the same. In some methods described herein, the first state of the molecular attribute has a first value, and the second state of the molecular attribute has a second value that is the same or different than the first state. By way of example, if the first state and the second state do not differ (or do not differ in a statistically significant manner), it may be determined that there is no change in the molecular attribute after subcutaneous administration. In some instances of the methods, the first state of the molecular attribute refers to a state of the molecular attribute prior to, or immediately upon administration to the subcutaneous space. The second state of the molecular attribute refers to a state of the molecular attribute in the subcutaneous space. The second state may refer to the state of the molecular attribute after it has been in the interface for a period of time, for example an incubation of seconds, minutes, or hours. The period of time may be specified prior to performing the method. In the method of some embodiments, the second state is the state of the therapeutic compound after it has been incubated in the interface for a specified period of time, for example, at least 1, 2, 3, 4, 5, 6, 7, 8, 9, 10, 15, 30, 45, or 60 minutes, or at least 1, 2, 3, 4, 5, 6, 7, 8, 9, 10, 11, 12, 18, 24, 36, 48, 60, 72, 84, 96, or 120 hours. For example, the specified period of time may be no more than 1, 2, 3, 4, 5, 6, 7, 8, 9, 10, 15, 30, 45, or 60 minutes, or no more than 1, 2, 3, 4, 5, 6, 7, 8, 9, 10, 11, 12, 18, 24, 36, 48, 60, 72, 84, 96, 120, 144, 168, 192, 216, or 240 hours. For example, the specified period of time may be 1-48 hours, 1-96 hours, 1-120 hours, 12-48 hours, 12-96 hours, 12-120 hours, 24-48 hours, 24-96 hours, or 24-120 hours. The incubation may be at a specified temperature, for example 20 °C, 25 °C, 33 °C (which is a typical human skin temperature), or 37 °C. In the method of some embodiments, the second state is the state of the therapeutic compound at a different temperature than in the first state. For example, the first state may be a temperature selected from: 20 °C, 25 °C, 33 °C, and 37 °C, and the second state may be a different temperature selected from: 20 °C, 25 °C, 33 °C, and 37 °C, so that the second state is a different temperature than the first state. By way of example, the detecting may be optical, or may comprise analytical testing of the therapeutic compound as described herein.

It is contemplated that in addition to effects on the bulk solution behavior, exposure to the skin environment may lead to changes in critical product quality attributes that affect drug quality and efficacy. Contact with the components of the injection site may lead to new interactions that facilitate modifications (e.g. oligomerization, redox reactions, conformational changes) that influence drug potency, immunogenicity, solubility, and stability. Accordingly, the method may comprise assessing and predicting potential changes for molecular attributes following sustained exposure to the *in vitro* mammalian skin environment. The simplicity of this approach permits methods described herein to be used as a platform to optimize formulations and explore strategies to maintain favorable product quality attributes.

In methods of detecting a change in a molecular attribute of some embodiments, the molecular attribute comprises at least one of acidic species, basic species, high molecular weight species, subvisible particle number, low molecular weight, middle molecular weight, glycosylation (such as non-glycosylated heavy chain or high mannose), non-heavy chain and light chain, deamidation, deamination, cyclization, oxidation, isomerization, fragmentation/clipping, N-terminal and C-terminal variants, reduced and partial species, folded structure, surface hydrophobicity, chemical modification, covalent bond, a C-terminal amino acid motif PARG, or a C-terminal amino acid motif PAR-Amide.

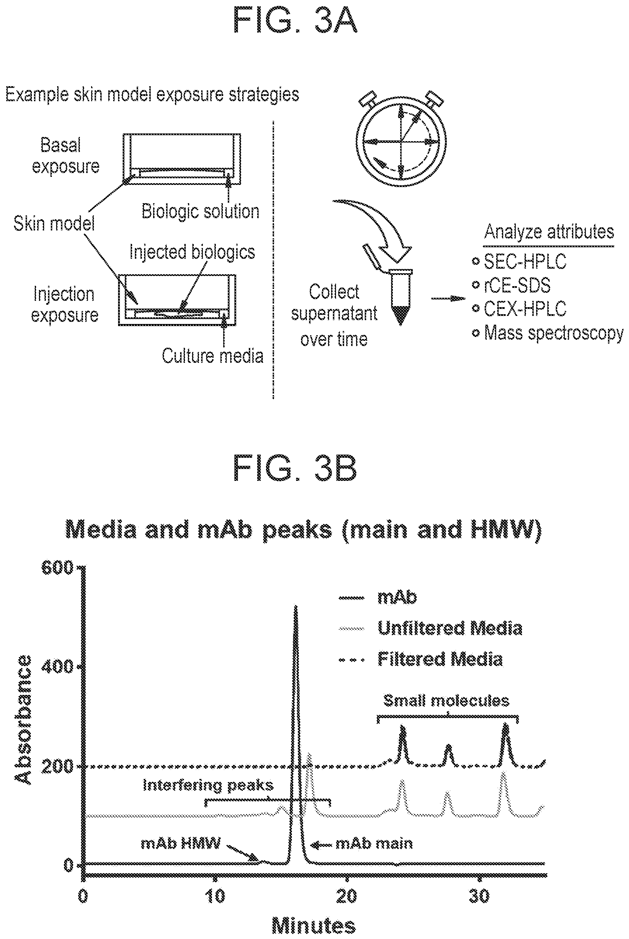

To examine how molecular attributes may change following incubation within the skin environment, therapeutic compounds may be exposed to *in vitro* mammalian skin models containing either the dermis or hypodermis. In methods of some embodiments, the exposure is done via the "basal exposure" or "injection exposure" as illustrated in Figure 3A. Basal exposure has the advantage of simplicity, but minor variants may be more difficult to distinguish from the major forms present in the bulk solution. Injection exposure more closely simulates subcutaneous injection, but may provide challenges if diffusion issues are present. In both strategies, the supernatant may be collected over time and evaluated using one or more analytical approaches (e.g., SE-HPLC, rCE-SDS, CEX-HPLC, HIAC, nrCE-SDS, mass spectrometry, mass spectrometry microscopy, and/or mass spectroscopy). An important consideration is the compatibility of the media for both the skin model and the analytical measurements. The media used must simultaneously sustain the skin model for the duration of the experiment and also not interfere with the measurement of interest. For example, the presence of high molecular weight (HMW) species and subvisible particles (SbVP) in the media may cause background interference with size exclusion chromatography, high performance liquid chromatography (SEC-HPLC), and high accuracy (HIAC) measurements. Accordingly, in some methods described herein, the media may be pre-filtered using centrifuge filters having a cutoff of no more than 50K Da (e.g., no more than 40K Da, 30K Da, or 25K Da). For example, to quantify the high molecular weight (HMW) species present using SE-HPLC, the media may be pre-filtered (e.g., using 50 kDa cutoff centrifuge filters) to reduce the presence of materials that interfere with the quantification as illustrated in the SE-HPLC chromatograms shown in Figure 3B. As an alternative to the media supplied by the vendors for the skin models, custom media may be used to more closely mimic the physiological conditions relevant to the skin environment during subcutaneous injection (e.g. addition of hyaluronic acid, use of blister/subcutaneous fluid). While these custom media may not be optimized for sustaining the skin models over long periods of time (> 2 days), they may provide insights that are more relevant to the interactions that occur immediately following subcutaneous injection (≤ 1 day timescales). These custom media may also provide reduced interference with the analytical measurement (the interference may be reduced compared to conventional commercial media). For example, the use of serum-free media may minimize or avoid serum proteins interfering with analytical measurements.

### Additional Aspects of Methods

For any of the methods described herein, it will be appreciated that the methods may further comprise, or may be further characterized by one or more of the additional aspects described herein.

For any of the methods described herein, providing the *in vitro* mammalian skin may comprise immobilizing the *in vitro* mammalian skin between a proximal substrate and a distal substrate. For example, the proximal substrate and the distal substrate each comprise glass, such as glass cover slips.

For any of the methods described herein, providing the *in vitro* mammalian skin may comprise providing the dermal layer of the *in vitro* mammalian skin on a porous substrate that comprises pores sized to accommodate diffusion of the therapeutic compound product. Administering the therapeutic compound product can thus comprise placing the porous substrate in fluid communication with a solution comprising the therapeutic compound product. Examples of porous substrates include polycarbonate, as well as polyester filter membrane. It is contemplated that porous substrates can permit diffusion of protein therapeutic product into the modeled subcutaneous space of the *in vitro* mammalian skin, obviating the need for administration by injection. As such in some embodiments, the dermal layer of the *in vitro* mammalian skin may be disposed on a porous substrate, and therapeutic compound product may be administered by simply contacting the porous substrate with solution comprising the therapeutic compound product, thus permitting the therapeutic compound to be administered to the interface between the dermal and epidermal layer. For example, the *in vitro* mammalian skin and porous substrate may be immersed in the solution. In some aspects, the method further comprises disposing the *in vitro* mammalian skin on the porous substrate.

For any of the methods described herein, administering may comprise pressing a needle comprising a cannula to a surface of the dermal layer, thus inserting the needle in the interface of the live *in vitro* mammalian skin. The method may further comprise disposing the therapeutic compound in the interface via the needle. For example, a specified volume of therapeutic compound (e.g., as part of a pharmaceutical composition) may be administered to the *in vitro* mammalian skin. In the method of some embodiments, the needle is pressed against the dermal surface at an acute angle, for example an angle of about 45 degrees or less. It is appreciated that for *in vitro* mammalian skin, pressing the needle at an acute angle can prevent or minimize damage or tearing of the skin. In the method of some embodiments, for example if the skin is relatively thick, the method may comprise pressing the needle against the dermal surface at a perpendicular or substantially perpendicular angle. In some methods described herein, the needle comprises an opening in fluid communication with the cannula, wherein administering comprises orienting the opening toward the dermal layer. In the methods of some embodiments, the needle remains disposed in the interface and therapeutic compound is administered until a sufficient volume of therapeutic compound has been administered to the interface. A sufficient volume of therapeutic compound may be determined by any suitable method, such as, for example, by measuring the bolus of material ejected from the needle at the site of injection. For example, the needle may remain disposed in the skin model for detecting the therapeutic compound product as described herein (e.g., by optical imaging), so that, in some embodiments, the injection site may be located and monitored throughout the injection period.

For any of the methods described herein, the *in vitro* mammalian skin may contain dermis and/or hypodermis. For such *in vitro* mammalian skin, the therapeutic compound may be administered into *in vitro* mammalian skin containing dermis and/or hypodermis (the layers typically exposed to therapeutic following subcutaneous injection, See Figure 1A), for example by injection. The reproducibility of this injection may be further enhanced by an injection apparatus that provides fine control of needle placement, penetration depth, and injection volume. Such an apparatus can permit injections to be targeted to specific positions and through different angles to accommodate the *in vitro* mammalian skin of varying dimensions.

For any of the methods described herein, administering may comprise soaking, diffusion, and/or transdermal administration of the therapeutic compound product through the dermal layer and/or into the hypodermal layer.

For any of the methods described herein, detecting may comprise optical imaging through the dermal layer. For any of the methods described herein, the detecting may comprise imaging and/or analytical testing.

For any of the methods described herein, the method may further comprise detecting a presence of level of subvisible particles in the solution after said administering.

It will be appreciated that any of the methods described herein may comprise analytical testing of the therapeutic compound (or composition comprising the therapeutic compound such as a pharmaceutical composition, or *in vitro* mammalian skin comprising the therapeutic compound). By way of example, the analytical testing may comprise one or more of mass spectrometry, chromatography, electrophoresis, spectroscopy, light obscuration, a particle method (such as nanoparticle/visible/micron-sized resonant mass or Brownian motion), analytical centrifugation, imaging or imaging characterization, immunoassay, SE-HPLC, rCE-SDS, CEX-HPLC, HIAC, nrCE-SDS, mass spectrometry microscopy, and/or mass spectroscopy. Techniques such as mass spectrometry microscopy may examine the distribution of chemical species in the *in vitro* mammalian skin. In some methods, analytical testing may be performed prior to the administration of the therapeutic compound and/or after administration of the therapeutic compound. For example, the "detecting" of any of the methods described herein may comprise analytical testing. For any of the methods described herein, the method may further comprise performing analytical testing on the therapeutic compound prior to the administering, for example to ascertain a baseline state of one or more molecular attributes prior to administration to the interface of the *in vitro* mammalian skin. For any of the methods described herein, analytical testing may be performed over a time course. For example, samples comprising the therapeutic compound may periodically be collected following administration of the therapeutic compound to the interface, and the samples may be analyzed with one or more analytical techniques as described herein. As such, the change (or lack of change) of one or more molecular attributes following administration to a skin environment may be determined.

Multiple approaches can be applied for detection after administration of the therapeutic compound. For methods described herein, the detection may characterize the distribution of therapeutic compound within skin and its ability to diffuse throughout tissue **(****Figure 1A****).** To resolve precipitation and potential accumulation within the *in vitro* mammalian skin, *in vitro* mammalian skin injected with the labeled therapeutic compound can be visualized using fluorescence imaging (e.g. multiphoton or confocal microscopy). For observing co-localization, the structures and cells of the skin models can also be fluorescently labeled. As methods described herein comprise disposing the therapeutic compound in the interface, the imaging techniques described herein are capable of imaging through the dermal layer. For example, the lasers used in conventional confocal microscopy may damage the *in vitro* mammalian skin, and the signal emitted by some fluorescent dyes may overlap with background fluorescence of the molecule/substance being visualized. Accordingly, in some methods described herein, detecting comprises imaging using multiphoton laser scanning microscopy (also referred to herein as "multiphoton microscopy"). Without being limited by any particular theory, multiphoton microscopy can create a gap between the excitation energy from autofluorescence and excitation energy from a fluorescent dye, thus minimizing overlap between the dye and background fluorescence. Moreover, multiphoton microscopy can minimize damage to *in vitro* mammalian skin tissue by pulsing photons from different directions so that the tissue does not experience as much energy. Multiphoton microscopy enables non-invasive deep imaging (up to about 1 mm) of thick living tissue specimens with high resolution. Tiling images with multiphoton microscopy also allows the observation of a wider field of view. These images provide direct qualitative insights into the bulk behavior of therapeutic formulations in the context of skin. Accordingly, in methods of some embodiments, the therapeutic compound is labeled. For example, in some embodiments, the therapeutic compound is labeled with a fluorescent dye that emits a signal that does not overlap with any background fluorescence (e.g., from collagen which is naturally autofluorescent). Exemplary fluorescent dyes include pH insensitive dyes, such as ALEXA^{™} Fluor 488. In other embodiments, detecting comprises the use of second-harmonic generation (SHG) imaging (also known as "frequency doubling"), which is a nonlinear optical imaging modality induced by a strong incident laser source such as multiphoton laser line. In SHG, two photons of the incident laser are mixed in the tissue and transformed into a single photon with doubled energy and frequency levels. SHG imaging is used in the art to visualize extracellular matrix (ECM) components and macromolecular structures, such as fibrillar collagen (see, e.g., Xie et al., Current Protocols in Cytometry, 6.33.1-6.33.11, July 2012). While not being bound to a particular theory, SHG may enable identification of the optimal focal plane for imaging. By way of example multiphoton laser wavelengths of about 850 nm, such as about 830-870 nm or about 840-860 nm can be suitable for generating a second harmonic collision for SHG imaging.

In some instances of methods described herein, after administration, a variety of quantitative analyses can be applied to the signal from fluorescently labeled therapeutic compound to characterize the distributions and diffusion properties of the biologics following injection. For example, (1) distributions of the injected biologics can be measured from the intensity profiles of the fluorescence signal, (2) fluorescence recovery after photobleaching (FRAP) can be applied to measure the diffusion coefficients for injected biologic solutions, and/or (3) measurements of the fluorescence signal in the media may provide insights into the diffusion kinetics of biologics exiting the injection site. Example schematics for these approaches are shown in FIG. 1A (lower panel "C").

For some instances of methods described herein, the detecting comprises CT scan, or magnetic resonance imaging. For some methods described herein, detecting comprises optical imaging. The optical imaging may be through the dermal layer. Accordingly, in any of the methods described herein, the therapeutic compound may be labeled. It will be appreciated that only a fraction of the administered therapeutic compound may be labeled in order to achieve suitable detection. Labeling may facilitate detection of the therapeutic compound and physiochemical and/or clinical characteristics of the therapeutic compound. As such, any of the methods described herein may further comprise labeling the therapeutic compound with a detectable moiety such as a fluorophore, fluorescent dye, radiolabel, or quantum dot. The therapeutic compound may be labeled, for example, using amine-reactive crosslinkers (e.g. via NHS-ester or SDP-ester coupled fluorophores).

To enhance the detection of therapeutic compound, background signal (for example signal from the *in vitro* mammalian skin obtained prior to administration of the therapeutic compound) may be subtracted. In the method of some embodiments, detecting comprises obtaining a background signal and subtracting the background signal.

### Additional Embodiments

Described herein are applications for using *in vitro* mammalian skin to examine the impact of the skin environment on therapeutic compounds. In some applications, therapeutic compounds (or pharmaceutical compositions comprising therapeutic compounds) are first exposed to models of the deeper layers of skin (dermis or hypodermis) to simulate the local conditions following subcutaneous injection. Some applications include examining the aggregation and distribution of biologics after injection into the skin environment. Some applications take a closer look at the physicochemical modifications and other molecular attribute changes that occur as drug products are sustained within the skin environment. Together, these approaches provide opportunities to gain insight into the drug-skin interactions relevant to subcutaneous injection. The simplicity of these *in vitro* approaches permits the implementation of screening strategies that can be used to optimize therapeutic compounds, formulations, and injection strategies to improve the quality, efficacy, and manufacturability of subcutaneously injected drug products. For example, the methods may be used to develop and select subcutaneous formulations of antibody protein products such as BiTE^{®} molecules comprising half-life extension moieties.

The use of *in vitro* mammalian skin to assess the impact of the skin environment on the therapeutic compound (or pharmaceutical composition comprising the therapeutic compound) may include two general steps: (1) drug exposure, (2) signal readout. The details of these two steps may be tailored for the particular application.

For example, an application of the methods described herein can comprise the assessment of bulk solution behavior, such as aggregation and diffusion. Therapeutic compounds that have been labeled (such as fluorescently labeled) are injected into skin models. Detection (e.g., imaging such as fluorescent imaging) is applied to directly observe the distribution and aggregation behavior of the therapeutic compounds within *in vitro* mammalian skin. Protein concentrations in the media outside *in vitro* mammalian skin may be monitored to characterize the diffusion of the biologic from the injection site. These approaches can be applied to screen strategies to minimize aggregation and achieve optimal distributions for drug bioavailability.

For example, an application can comprise the assessment of attribute changes (e.g., high molecular weight (HMW), subvisible particles, and/or chemical changes). *In vitro* mammalian skin may be used to determine changes in the molecular attributes of therapeutic compounds that occur following skin exposure. Analytical approaches (e.g., size exclusion chromatography (SEC-HPLC), capillary electrophoresis, cation/anion exchange, mass spectroscopy, HIAC particle counting) are applied to quantify changes in the molecular attributes over time. Therapeutic compounds and formulations may be screened and optimized to maintain favorable attribute levels and conditions.

### EXAMPLES

### EXAMPLE 1: Bulk solution behavior (e.g., aggregation and diffusion)

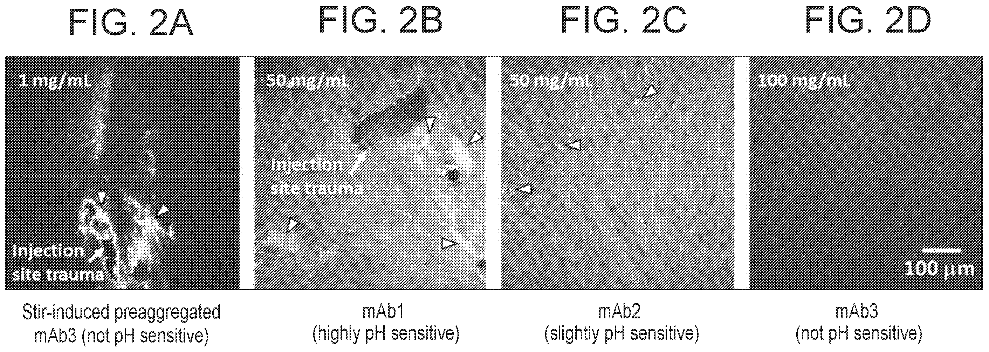

To demonstrate how *in vitro* mammalian skin can be used to resolve protein aggregation within the context of skin, several fluorescently labeled monoclonal antibody (mAb) solutions with varying pH-sensitive propensities to aggregate were injected into skin models and imaged using multiphoton microscopy. As a positive control, pre-aggregated solutions of mAb3 (prepared by applying a stirring stress) were injected into the skin models **(****FIG. 2A**). Pre-aggregated mAb3 was visualized as bright spots that localized near the injection site (which can be identified by the black areas of the image that reveal tissue trauma from the injection). Diffuse fluorescence outside of these areas were not observed, indicating that the aggregation was not reversible and limited the ability for the mAb to diffuse throughout the tissue. To determine whether aggregation can be observed within the skin model, mAb1, a mAb known to strongly aggregate at neutral pH, was injected into the skin model (**FIG**. **2B**). Bright spots were observed that primarily localized near the injection site, confirming that mAb1 aggregated within the skin model following injection. Diffuse fluorescence was observed, showing that some mAb1 remained unaggregated and was able to diffuse throughout the skin model. A mAb with mild pH sensitivity, mAb2, was also injected into the skin model (**FIG. 2C**). The presence of diffuse scatter and fewer bright spots indicate that this mAb only mildly aggregated upon injection. Lastly, when a mAb without pH sensitivity, mAb3, was injected into the skin model, no signs of aggregation were observed (**FIG. 2D**). The results summarized in FIGs. 2A-D demonstrate how skin models can be used to screen biologic solutions for their propensity to aggregate upon injection into the skin environment. Ongoing developments will enable the application of the quantitative analyses shown in FIG. 1A (lower panel "C").

### EXAMPLE 2: Molecular attribute changes

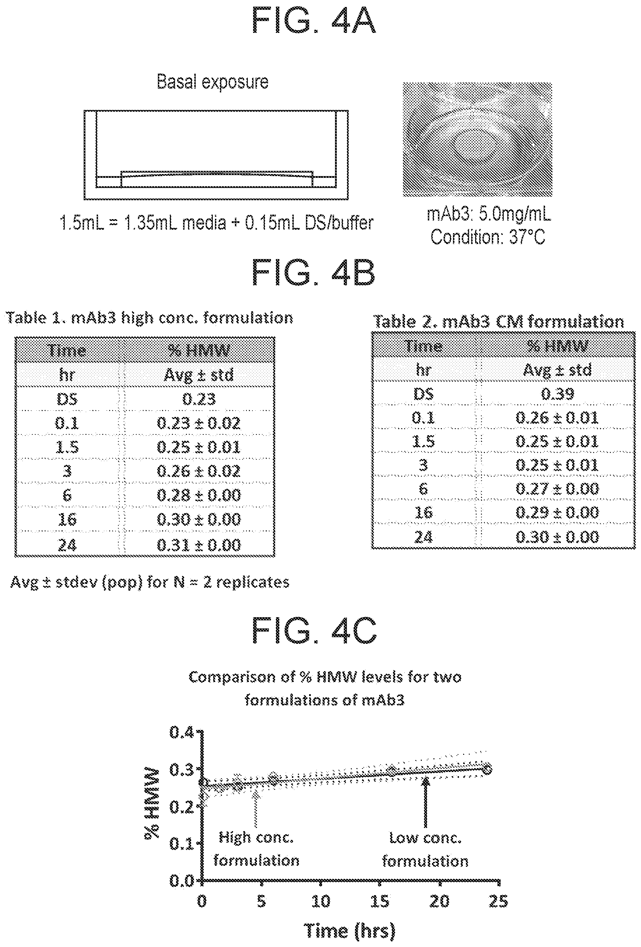

To demonstrate how skin models may be used to assess attribute changes due to skin exposure, experiments were performed comparing the levels of high molecular weight (HMW) species and subvisible particles (SbVP) for two formulations of the same therapeutic protein (mAb3). The concentration of mAb3 in a higher concentration formulation (100 mg/mL, "HC") was double the concentration present in a lower concentration formulation (50 mg/mL, "LC"). Furthermore, the two formulations had different buffer compositions (lactate buffer and acetate buffer for the HC and LC formulations, respectively). Both formulations were diluted to the same final concentration (5.0 mg/mL) using media and exposed to the skin models using "basal exposure" as illustrated in **FIG. 4A****.** The media used for the dilution was filtered using 50 kDa cutoff centrifuge filters to remove proteins with sizes that interfered with the quantification of main peak and HMW peak species (**FIG. 3B**). The filtered media did not exhibit background interference with SEC-HPLC for canonical antibodies, nor for BiTE^{®} molecules comprising half-life extension moieties. Accordingly, it was observed that filtering the media permitted the skin models described herein to characterize HMW and SbVP change during exposure. For HIAC analysis, the filtered media also exhibited manageable background.

Small aliquots of the exposed biologic solution were collected over a timecourse that ranged from 0.1 to 24 hrs and analyzed using size-exclusion HPLC. The % HMW species detected are compared in the tables shown in **FIG. 4B****.** For these product lots, the initial level of HMW for the LC formulation drug substance (DS) was slightly higher (0.39%) compared to the HC formulation (0.23%), however, upon dilution, the levels of HMW became comparable (0.26 and 0.23, respectively). For both formulations, the levels of HMW slightly increased over the span of 24 hours, but did so in a similar manner, reaching a final level of 0.30% and 0.31% for the LC and HC formulations, respectively. The absence of differences in the HMW formation rates between the LC and HC formulations (**FIGs**. **4B** and **4C**) indicates that the HC formulation poses no increased risk of HMW formation as compared to the LC formulation. This agreement is underscored by the overlap between the 95% confidence intervals for the fits (dashed and dotted lines in **FIG. 4C**). Note that the levels of HMW are likely to be sensitive to concentration, which would diminish as the drug product enters circulation and becomes diluted from the injection site area. An example of this effect is seen in the LC formulation, where the levels drop from 0.39% in the DS to 0.26% following the 10-fold dilution (50 to 5 mg/mL, **FIG. 4B****,** Table 2). The exposure strategy applied here may be used to reveal attribute changes for biologics that are sustained within the injection site or represent scenarios of what may occur if proper drug distribution was inhibited. These results showing similarities between the two formulations suggest no additional risk of HMW species for the higher concentrated HC formulation relative to the lower concentrated LC formulation when administered at the same dose.

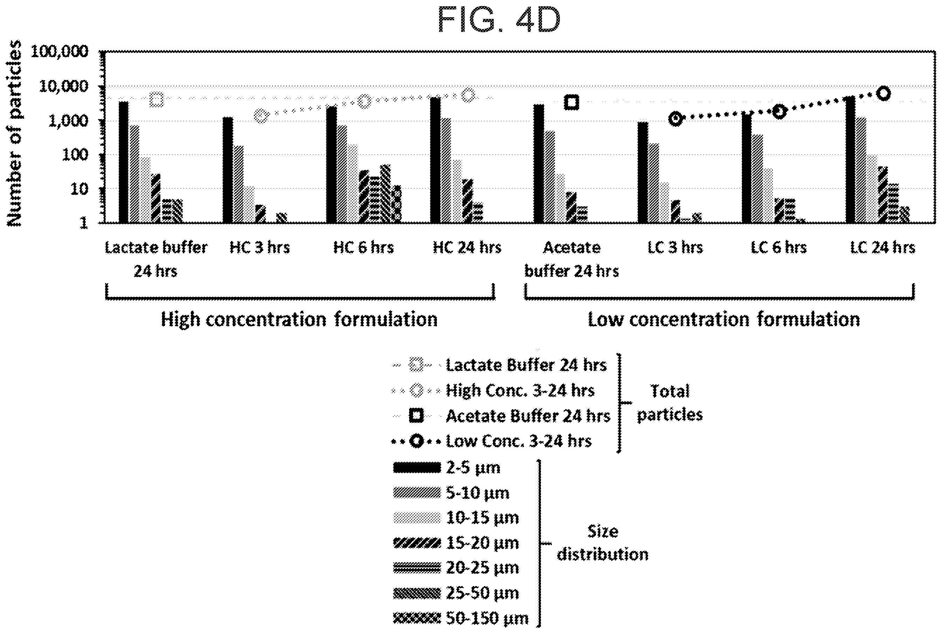

The levels of subvisible particles (2-150 µm SbVP) were monitored following basal exposure as illustrated in **FIG. 4A****.** Media was collected over a span of 24 hrs and the subvisible particles were quantified using a HIAC liquid particle counter. The number of particles detected at 3, 6, and 24 hrs for both the HC and LC formulations are indicated by the symbols in **FIG. 4D****.** Similar increases were detected for both formulations. Notably, the number of particles detected after 24 hrs for skin models exposed to the formulation buffers alone without biologics (square symbols and gray dashed lines) were similar to that reached for skin models exposed to the biologic formulations after 24 hrs. This suggests that the majority of these particles are from the skin model and/or media, and unrelated to the biologic formulations. Particle size distributions are shown below each datapoint and are similar for skin models exposed to both formulations. The agreement of these results suggests that the impact of the skin environment on these two formulations was largely comparable and no additional risk of subvisible particle formation was found between the two formulations of mAb3.

### Example 3: In vitro aggregation and molecular attribute changes in additional therapeutic proteins

To demonstrate the use of *in vitro* mammalian skin models to examine various factors of therapeutic protein solutions that may impact the extent of aggregation and formation of HMW species due to contact with the skin environment, skin models were exposed to a variety of therapeutic proteins with different targets, therapeutic protein modalities, buffer compositions, pH, and initial attribute levels. Four different therapeutic proteins were examined: (1) an antibody protein product with a half-life extension moiety (Therapeutic Protein 1, TP1), (2-3) two antibody protein product molecules, each with specificities to two different targets (Therapeutic Protein 2 and Therapeutic Protein 3, TP2 and TP3, respectively), and (4) a mAb known to strongly aggregate at neutral pH (mAb1). For Therapeutic Protein 1, two different formulations were used, a high concentration (HC) formulation (20 mg/mL) and low concentration (LC) formulation (1 mg/mL), each with different buffer compositions, but formulated at pH 4.2. Furthermore, for the HC formulation of Therapeutic Protein 1, the protein solutions were prepared with two different approaches: unstressed, and stressed with 5X freeze/thaws (F/T) cycles, which was known to elevate the levels of HMW species. The other proteins (Therapeutic Protein 2, Therapeutic Protein 3, mAb1) were formulated with different protein concentrations (0.8, 2.5, and 20 mg/mL, respectively), buffer compositions, pH (7.0, 6.0, 5.2, respectively), and were prepared without any additional stresses. *In vitro* mammalian skin models were basally exposed to these protein solutions by the addition of these protein solutions into the media, resulting in final dilution factors of 10X or 2X for the protein solutions from their initial formulated concentrations.

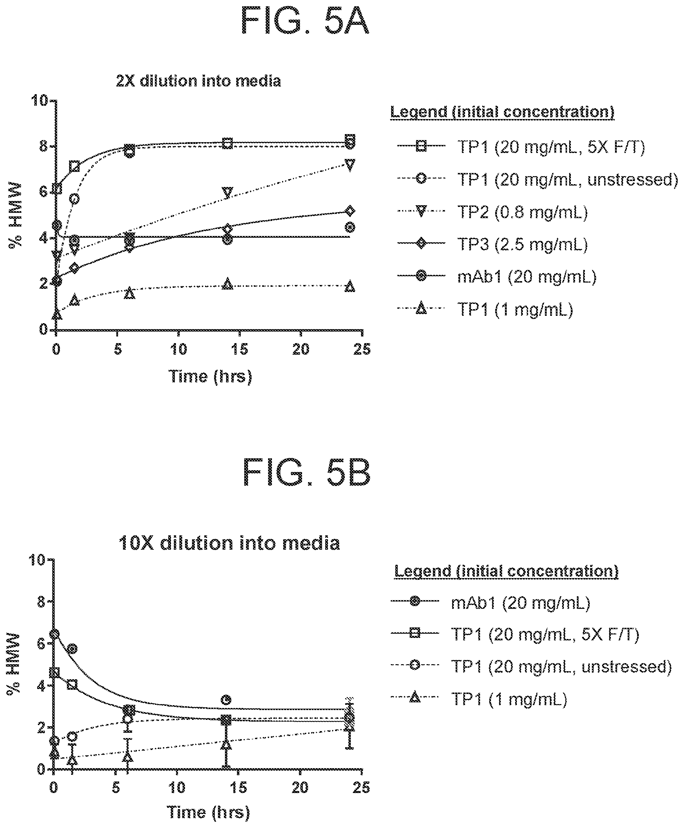

*In vitro* mammalian skin models were incubated for 24 hrs with small aliquots collected from the media over a period of 24 hrs to monitor the levels of HMW species and SbVPs. The % HMW species detected for these protein solutions when exposed at 2X dilution and 10X dilution are shown in Figure 5A and Figure 5B, respectively. At both the 2X and 10X dilutions, the HC formulation Therapeutic Protein 1 prepared with the 5X F/T stress initially had higher levels of % HMW compared to unstressed samples of the same formulation, but plateaued to similar levels after about 6 hrs. At the 2X dilution, the HC formulations of Therapeutic Protein 1 plateaued to a higher level of % HMW (8%) compared to the LC formulation of Therapeutic Protein 1 (2%). However, at 10X dilution, the HC formulations of Therapeutic Protein 1 plateaued to similar levels as the LC formulation of Therapeutic Protein 1 (2%), suggesting that the final levels of % HMW formed for Therapeutic Protein 1 are concentration dependent. The % HMW increased for both Therapeutic Protein 2 and Therapeutic Protein 3 at 2X dilution, with Therapeutic Protein 2 increasing at a slightly higher rate, despite being at a lower concentration (0.8 vs 2.5 mg/mL). This suggests that in some cases, the physicochemical nature of the proteins and formulation buffers may more greatly impact the formation of HMW species than the protein concentrations. The % HMW detected for mAb1, which is known to aggregate at neutral pH, decreased initially and plateaued to 4% at 2X dilution, and 2% at 10X dilution. Notably, the % HMW for most proteins generally increased when exposed to the skin model and media, with the exception of mAb1 at both dilutions and HC Therapeutic Protein 1 (5X F/T) at the 10X dilution, suggesting that the levels of % HMW formed within the skin environment may be dependent on both the nature of the multimerization and the concentration within the skin environment.

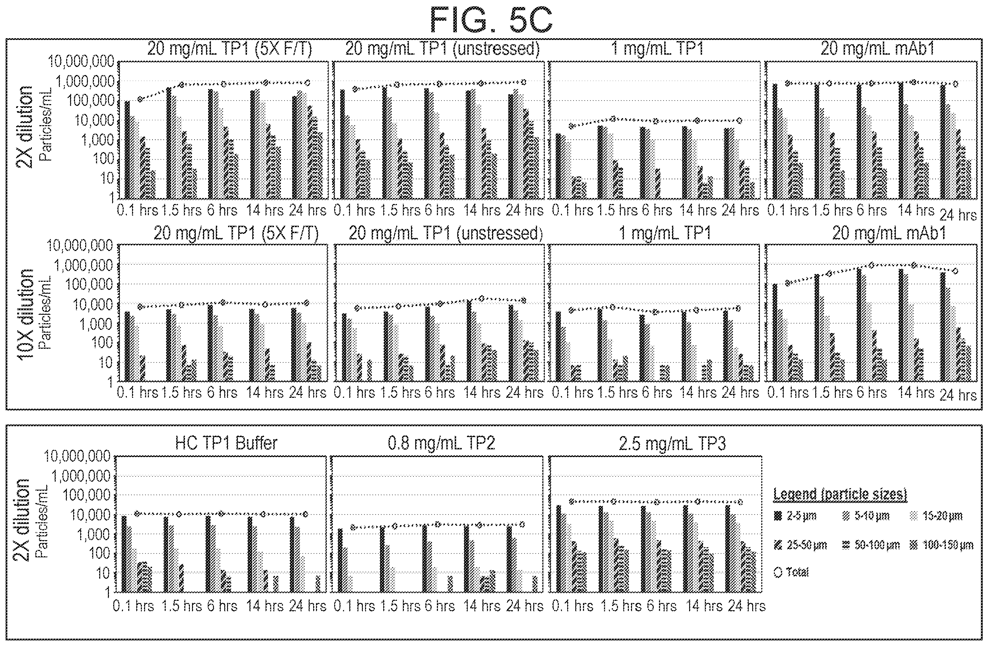

The levels of subvisible particles (2-150 µm SbVP) present in the media were quantified using a HIAC liquid particle counter after the therapeutic protein solutions were incubated with the skin models for 24 hrs. The particle concentrations are shown in Figure 5C. For skin models exposed to the 5X F/T and unstressed 20 mg/mL (HC) formulations of Therapeutic Protein 1 at a 2X dilution, approximately 1,000,000 particles/mL were detected by 1.5 hrs, and the proportion of larger sized particles increased over the span of 24 hrs. The number of particles detected were 100-fold lower (approximately 10,000 particles/mL) for the 1 mg/mL (LC) Therapeutic Protein 1 at the same 2X dilution. However, when skin models were exposed to the 20 mg/mL (HC) Therapeutic Protein 1 formulations at a 10X dilution, similar numbers of particles (approximately 10,000 particles/mL) were measured between both the HC and LC formulations of Therapeutic Protein 1. Without being limited by theory, this suggests that the formation of particles for Therapeutic Protein 1 is strongly concentration dependent. The media from skin models exposed to the formulation butter for HC Therapeutic Protein 1 had approximately 10,000 particles/mL present, indicating that this may be the baseline level of SbVP formed from the skin model/media system when exposed to the formulation buffer. Hence, without being limited by theory, for 20 mg/mL (HC) Therapeutic Protein 1, a 10X dilution within the skin environment may be sufficient for reducing SbVP formation due to local interactions at the injection site. For skin models exposed to mAb1, which was known to strongly aggregate at neutral pH, aggregation remained high, as approximately 1,000,000 particles were detected at both the 2X and 10X dilutions. The levels of SbVP detected in the media for skin models exposed to Therapeutic Protein 2 and Therapeutic Protein 3 formulations at 2X dilutions were approximately 3000 particles/mL and 50000 particles/mL, respectively, and remained fairly stable across 24 hrs. Overall, these results suggest that the propensities to form SbVP within the skin environment may vary for different therapeutic compound formulations and may be concentration dependent depending on the nature of the aggregation. This experiment demonstrates how *in vitro* mammalian skin models may be used to screen formulation conditions and delivery strategies to reduce the risk of aggregation at the injection site.

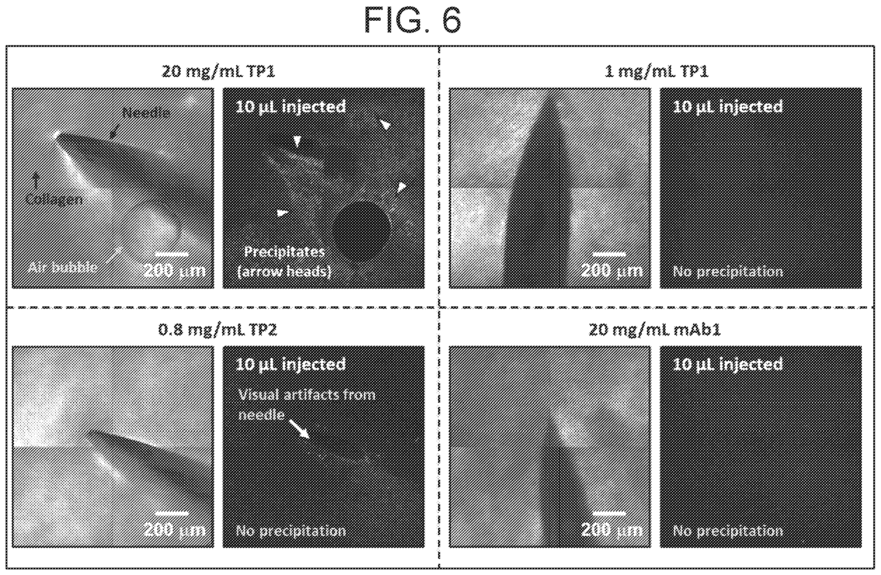

To resolve *in vitro* aggregation of therapeutic proteins injected into *in vitro* mammalian skin models, proteins were fluorescently labeled, injected into *in vitro* mammalian skin models, and imaged using multiphoton microscopy as shown in Figures 1A-1B. In this setup, the injection needle was inserted into the skin model and remained embedded for the duration of the experiment. This permitted the injection site to be located and monitored throughout the injection process. A syringe pump was used to control the injection volume and multiphoton microscopy permitted the imaging of both the collagen structure within the skin model and the fluorescently labeled proteins as they were being injected into the skin models. Images of the injection site after 10 µL of various fluorescently labeled proteins were injected are shown in Figure 6. No signs of aggregation were observed near the injection site for the unstressed 20 mg/mL (HC) formulation of Therapeutic Protein 1, 1 mg/mL (LC) formulation of Therapeutic Protein 1, or 0.8 mg/mL formulation of Therapeutic Protein 2, however significant levels of aggregation (bright spots) were observed for the 20 mg/mL mAb1, which was expected to aggregate strongly at neutral pH. This experiment demonstrates how *in vitro* mammalian skin models can be used with fluorescent imaging strategies to detect *in vitro* aggregation within *in vitro* mammalian skin models.

The Examples described herein illustrate, *inter alia,* how methods described herein may use in vitro mammalian skin to investigate the impact of the skin environment on the physicochemical properties of subcutaneously injected biologics. The simplicity of the methods may facilitate screening strategies to better understand and optimize drug products that are delivered through skin. Additional uses of methods described herein are contemplated, including: (1) the use of skin models based on the hypodermis layer (which may be more relevant to SubQ exposure), (2) development of an apparatus to improve injection consistency, (3) application of more quantitative analyses of drug distributions and attribute changes. Lastly, the use of skin models with larger formats may be useful for simulating the injection of biologic solutions at therapeutically relevant volumes, which would enable the testing and optimization of injection strategies (e.g. addition of hyaluronidase) and injection devices.

### References

The following documents are incorporated by reference in their entireties herein.
1. Turner MR, Balu-iyer S V. Challenges and Opportunities for the Subcutaneous Delivery of Therapeutic Proteins. J Pharm Sci. 2018;107(5):1247-1260. doi:10.1016/j.xphs.2018.01.007
2. Poumay Y, Dupont F, Marcoux S, Leclercq-Smekens M, Hérin M, Coquette A. A simple reconstructed human epidermis: Preparation of the culture model and utilization in in vitro studies. Arch Dermatol Res. 2004;296(5):203-211. doi:10.1007/s00403-004-0507-y
3. De Wever BDE, Petersohn D, Mewes KR. Overview of human three-dimensional (3D) skin models used for dermal toxicity assessment (Part 1). Household and Personal Care Today. 2013;8(1):18-22.
4. MatTek tissue webpage. https://www.mattek.com/product-category/tissue-models/
5. Rosdy M, Clauss LC. Terminal epidermal differentiation of human keratinocytes grown in chemically defined medium on inert filter substrates at the air-liquid interface. J Invest Dermatol. 1990;95(4):409-414. doi:10.1111/1523-1747.ep12555510
6. Ng KW, Khor HL, Hutmacher DW. In vitro characterization of natural and synthetic dermal matrices cultured with human dermal fibroblasts. Biomaterials. 2004;25(14):2807-2818. doi:10.1016/j.biomaterials.2003.09.058
7. Louis F, Pannetier P, Souguir Z, et al. A biomimetic hydrogel functionalized with adipose ECM components as a microenvironment for the 3D culture of human and murine adipocytes. Biotechnol Bioeng. 2017;114(8):1813-1824. doi:10.1002/bit.26306
8. Bell E, Sher S, Hull B, et al. The reconstitution of living skin. J Invest Dermatol. 1983;(81):2-10.
9. Schmidt FF, Nowakowski S, Kluger PJ. Improvement of a Three-Layered in vitro Skin Model for Topical Application of Irritating Substances. Front Bioeng Biotechnol. 2020;8(May):1-11. doi:10.3389/fbioe.2020.00388
10. Alépée N, Tornier C, Robert C, et al. A catch-up validation study on reconstructed human epidermis (Skin Ethic™ RHE) for full replacement of the Draize skin irritation test. Toxicol Vitr. 2010;24(1):257-266. doi:10.1016/j.tiv.2009.08.024
11. European THE, Council P, The OF, et al. REGULATION (EC) No 1223/2009 OF THE EUROPEAN PARLIAMENT AND OF THE COUNCIL of 30 November 2009 on cosmetic products. Off J Eur Union L. 2009;342(59).

The use of the terms "a" and "an" and "the" and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

The terms "patient" and "subject" are used interchangeably herein. Generally, these terms will be understood to refer to humans. In the methods of some embodiments, the patient or subject is a human.

Recitation of ranges of values herein are merely intended to serve as a shorthand method of referring individually to each separate value falling within the range and each endpoint, unless otherwise indicated herein, and each separate value and endpoint is incorporated into the specification as if it were individually recited herein.

All methods described herein can be performed in any suitable order unless otherwise indicated herein or otherwise clearly contradicted by context. The use of any and all examples, or exemplary language (e.g., "such as") provided herein, is intended merely to better illuminate the disclosure and does not pose a limitation on the scope of the disclosure unless otherwise claimed. No language in the specification should be construed as indicating any non-claimed element as essential to the practice of the disclosure.

Preferred embodiments of this disclosure are described herein, including the best mode known to the inventors for carrying out the disclosure.

## Claims

1. A method of selecting a condition of administration for a therapeutic compound for subcutaneous administration, the method comprising;
providing a live *in vitro* mammalian skin comprising a dermal layer and epidermal layer that define an interface therebetween;
administering a therapeutic compound to the interface under a condition of administration; wherein the therapeutic compound is selected from an antibody, an antigen-binding antibody fragment, an antibody protein product, a Bi-specific T cell engager molecule, and a bispecific antibody;
detecting a physiochemical and/or clinical characteristic of the therapeutic compound in the interface; and
selecting or rejecting the condition of administration for further development based on the detected physiochemical and/or clinical characteristic of the therapeutic compound.

2. The method of claim 1, wherein the condition of administration comprises at least one of formulation, concentration, volume, viscosity, a molecular attribute of said therapeutic compound, route of administration, and/or administration apparatus or method of using an administration apparatus.

3. The method of claim 1 or claim 2, wherein the physiochemical and/or clinical characteristic comprises bioavailability, pharmacokinetic property, aggregation, precipitation, distribution, diffusion rate, retention and/or absorption, and wherein the therapeutic compound acquired the physiochemical and/or clinical characteristic after said administering.

4. The method of any one of claims 1-3, wherein the therapeutic compound comprises a molecular attribute.

5. The method of claim 4, wherein the therapeutic compound comprises a first state of the molecular attribute and the method further comprises detecting a second state of the molecular attribute acquired after said administering.

6. The method of claim 4 or claim 5, wherein the molecular attribute comprises at least one of acidic species, basic species, high molecular weight species, subvisible particle number, low molecular weight, middle molecular weight, glycosylation (such as non-glycosylated heavy chain or high mannose), non-heavy chain and light chain, deamidation, deamination, cyclization, oxidation, isomerization, fragmentation/clipping, N-terminal and C-terminal variants, reduced and partial species, folded structure, surface hydrophobicity, chemical modification, covalent bond, a C-terminal amino acid motif Pro-Ala-Arg-Gly (PARG), or a C-terminal amino acid motif Pro-Ala-Arg (PAR)-Amide.

7. The method of any one of claims 1-6, wherein the *in vitro* mammalian skin further comprises a hypodermis that defines a subcutaneous space within the interface.

8. The method of claim 7, wherein administering comprises:
pressing a needle comprising a cannula to a surface of the dermal layer, thereby inserting the needle in the interface of the live *in vitro* mammalian skin; and
disposing the therapeutic compound product in the subcutaneous space via the needle.

9. The method of any one of claims 1-8, wherein providing the *in vitro* mammalian skin comprises immobilizing the *in vitro* mammalian skin between a proximal substrate and a distal substrate.

10. The method of any one of claims 1-9, wherein the *in vitro* mammalian skin is an *in vitro* human skin.

11. The method of any one of claims 1-10, wherein the detecting comprises optical imaging.

12. The method of claim 11, wherein the optical imaging comprises multiphoton laser scanning microscopy and/or second-harmonic generation (SHG) imaging.

13. The method of claim 11 or 12, wherein administering the therapeutic compound comprises disposing the therapeutic compound in the subcutaneous space via a needle, and wherein the needle remains disposed in the subcutaneous space during said imaging.

14. The method of any one of claims 1-13, wherein the live *in vitro* mammalian skin is provided in media, wherein the media has been filtered prior to said providing.

15. The method of any one of claims 4-6, wherein the molecular attribute is detected by size exclusion chromatography, high performance liquid chromatography (SEC-HPLC) and a high accuracy (HIAC) particle counter.

## Patentansprüche

1. Verfahren zum Auswählen einer Verabreichungsbedingung für eine therapeutische Verbindung zur subkutanen Verabreichung, wobei das Verfahren Folgendes umfasst:
Bereitstellen einer lebenden *in* vitro-Säugetierhaut, die eine dermale Schicht und eine epidermale Schicht umfasst, die eine dazwischenliegende Grenzschicht definieren;
Verabreichen einer therapeutischen Verbindung an die Grenzschicht unter einer Verabreichungsbedingung;
wobei die therapeutische Verbindung ausgewählt ist aus einem Antikörper, einem Antigen-bindenden Antikörperfragment, einem Antikörperproteinprodukt, einem bispezifischen T-Zell-Aktivator-Molekül und einem bispezifischen Antikörper;
Nachweisen einer physikochemischen und/oder klinischen Eigenschaft der therapeutischen Verbindung in der Grenzschicht; und
Auswählen oder Verwerfen der Verabreichungsbedingung für die weitere Entwicklung auf Grundlage der festgestellten physikochemischen und/oder klinischen Eigenschaften der therapeutischen Verbindung.

2. Verfahren nach Anspruch 1, wobei die Verabreichungsbedingung mindestens eine der folgenden umfasst: Formulierung, Konzentration, Volumen, Viskosität, ein molekulares Merkmal der therapeutischen Verbindung, Verabreichungsweg und/oder Verabreichungsvorrichtung oder Verfahren zur Verwendung einer Verabreichungsvorrichtung.

3. Verfahren nach Anspruch 1 oder Anspruch 2, wobei die physikochemische und/oder klinische Eigenschaft Bioverfügbarkeit, eine pharmakokinetische Eigenschaft, Aggregation, Präzipitation, Verteilung, Diffusionsrate, Retention und/oder Absorption umfassen, und wobei die therapeutische Verbindung die physikochemischen und/oder klinischen Eigenschaften nach der Verabreichung erlangt hat.

4. Verfahren nach einem der Ansprüche 1-3, wobei die therapeutische Verbindung ein molekulares Merkmal umfasst.

5. Verfahren nach Anspruch 4, wobei die therapeutische Verbindung einen ersten Zustand des molekularen Merkmals umfasst und das Verfahren ferner das Nachweisen eines zweiten Zustands des molekularen Merkmals umfasst, der nach der Verabreichung erlangt wird.

6. Verfahren nach Anspruch 4 oder Anspruch 5, wobei das molekulare Merkmal mindestens eines der folgenden umfasst: saure Spezies, basische Spezies, Spezies mit hohem Molekulargewicht, subvisible Partikelzahl, niedriges Molekulargewicht, mittleres Molekulargewicht, Glykosylierung (wie etwa nicht-glykosylierte schwere Kette oder hoher Mannose-Gehalt), nichtschwere Kette und leichte Kette, Deamidierung, Deaminierung, Zyklisierung, Oxidation, Isomerisierung, Fragmentierung/Clipping, N-terminale und C-terminale Varianten, reduzierte und partielle Spezies, gefaltete Struktur, Oberflächenhydrophobie, chemische Modifikation, kovalente Bindung, ein C-terminales Aminosäuremotiv Pro-Ala-Arg-Gly (PARG) oder ein C-terminales Aminosäuremotiv Pro-Ala-Arg (PAR)-Amid.

7. Verfahren nach einem der Ansprüche 1-6, wobei die *in* vitro-Säugetierhaut ferner eine Hypodermis umfasst, die einen subkutanen Raum innerhalb der Grenzschicht definiert.

8. Verfahren nach Anspruch 7, wobei das Verabreichen Folgendes umfasst:
Drücken einer Nadel, die eine Kanüle umfasst, auf eine Oberfläche der Hautschicht, wodurch die Nadel in die Grenzfläche der lebenden *in* vitro-Säugerhaut eingeführt wird; und
Einbringen des therapeutischen Verbindungsprodukts in den subkutanen Raum mittels der Nadel.

9. Verfahren nach einem der Ansprüche 1-8, wobei das Bereitstellen der *in vitro*-Säugetierhaut das Immobilisieren der *in vitro*-Säugetierhaut zwischen einem proximalen Träger und einem distalen Träger umfasst.

10. Verfahren nach einem der Ansprüche 1-9, wobei die *in vitro*-Säugetierhaut eine *in vitro* menschliche Haut ist.

11. Verfahren nach einem der Ansprüche 1-10, wobei das Nachweisen eine optische Bildgebung umfasst.

12. Verfahren nach Anspruch 11, wobei die optische Bildgebung Multiphotonen-Laser-Scanning-Mikroskopie und/oder Frequenzverdopplungs (SHG)-Bildgebung umfasst.

13. Verfahren nach Anspruch 11 oder 12, wobei das Verabreichen der therapeutischen Verbindung das Einbringen der therapeutischen Verbindung in den subkutanen Raum mittels einer Nadel umfasst, und wobei die Nadel während der Bildgebung im subkutanen Raum verbleibt.

14. Verfahren nach einem der Ansprüche 1-13, wobei die lebende *in vitro*-Säugetierhaut in einem Medium bereitgestellt wird, wobei das Medium vor der Bereitstellung gefiltert wurde.

15. Verfahren nach einem der Ansprüche 4-6, wobei das molekulare Merkmal durch Größenausschlusschromatographie, Hochleistungsflüssigkeitschromatographie (SEC-HPLC) und einen hochgenauen (HIAC) Partikelzähler nachgewiesen wird.

## Revendications

1. Procédé de sélection d'une condition d'administration d'un composé thérapeutique destiné à une administration sous-cutanée, le procédé comprenant :
la fourniture d'une peau de mammifère vivante *in vitro* permettant de fournir une couche dermique et une couche épidermique qui définissent une interface entre elles ;
l'administration d'un composé thérapeutique à l'interface dans des conditions d'administration ;
dans lequel le composé thérapeutique est choisi parmi un anticorps, un fragment d'anticorps se liant à un antigène, un produit protéique anticorps, une molécule de liaison aux cellules T bispécifiques et un anticorps bispécifique ;
la détection d'une caractéristique physicochimique et/ou clinique du composé thérapeutique dans l'interface ; et
la sélection ou le rejet de la condition d'administration pour un développement ultérieur sur la base de la caractéristique physicochimique et/ou clinique détectée du composé thérapeutique.

2. Procédé selon la revendication 1, dans lequel la condition d'administration comprend au moins l'un parmi la formulation, la concentration, le volume, la viscosité, une propriété moléculaire dudit composé thérapeutique, la voie d'administration et/ou l'appareil d'administration ou le mode d'emploi à l'aide d'un appareil d'administration.

3. Procédé selon la revendication 1 ou la revendication 2, dans lequel la caractéristique physicochimique et/ou clinique comprend la biodisponibilité, la propriété pharmacocinétique, l'agrégation, la précipitation, la distribution, la vitesse de diffusion, la rétention et/ou l'absorption, et dans lequel le composé thérapeutique a acquis la caractéristique physicochimique et/ou clinique après ladite administration.

4. Procédé selon l'une quelconque des revendications 1 à 3, dans lequel le composé thérapeutique comprend un attribut moléculaire.

5. Procédé selon la revendication 4, dans lequel le composé thérapeutique comprend un premier état de l'attribut moléculaire et le procédé comprend en outre la détection d'un deuxième état de l'attribut moléculaire acquis après ladite administration.

6. Procédé selon la revendication 4 ou la revendication 5, dans lequel l'attribut moléculaire comprend au moins l'un des éléments suivants : les espèces acides, les espèces basiques, les espèces de poids moléculaire élevé, le nombre de particules subvisibles, le faible poids moléculaire, le poids moléculaire moyen, la glycosylation (telle que la chaîne lourde non glycosylée ou le mannose élevé), la chaîne non lourde et la chaîne légère, la désamidation, la désamination, la cyclisation, l'oxydation, l'isomérisation, la fragmentation ou le clivage, les variants N-terminaux et C-terminaux, les espèces réduites et partielles, la structure repliée, l'hydrophobicité de surface, la modification chimique, la liaison covalente, un motif d'acide aminé C-terminal Pro-Ala-Arg-Gly (PARG) ou un motif d'acide aminé C-terminal Pro-Ala-Arg (PAR)-Amide.

7. Procédé selon l'une quelconque des revendications 1 à 6, dans lequel la peau de mammifère in vitro comprend en outre un hypoderme qui définit un espace sous-cutané au sein de l'interface.

8. Procédé selon la revendication 7, dans lequel l'administration comprend :
le fait d'appuyer une aiguille comprenant une canule sur une surface de la couche dermique, ce qui permet d'insérer l'aiguille dans l'interface de la peau de mammifère vivante *in vitro* ; et
le fait de disposer le produit composé thérapeutique dans l'espace sous-cutané par l'intermédiaire de l'aiguille.

9. Procédé selon l'une quelconque des revendications 1 à 8, dans lequel la fourniture de la peau de mammifère in vitro comprend l'immobilisation de la peau de mammifère in vitro entre un substrat proximal et un substrat distal.

10. Procédé selon l'une quelconque des revendications 1 à 9, dans lequel la peau de mammifère in vitro est une peau humaine in vitro.

11. Procédé selon l'une quelconque des revendications 1 à 10, dans lequel la détection comprend une imagerie optique.

12. Procédé selon la revendication 11, dans lequel l'imagerie optique comprend une microscopie à balayage laser multi-photonique et/ou une imagerie par génération de deuxième harmonique (SHG).

13. Procédé selon la revendication 11 ou la revendication 12, dans lequel l'administration du composé thérapeutique comprend la mise en place du composé thérapeutique dans l'espace sous-cutané via une aiguille, et
dans lequel l'aiguille reste en place dans l'espace sous-cutané pendant ladite imagerie.

14. Procédé selon l'une quelconque des revendications 1 à 13, dans lequel la peau de mammifère vivante in vitro est fournie dans un milieu, dans lequel le milieu a été filtré avant ladite fourniture.

15. Procédé selon l'une quelconque des revendications 4 à 6, dans lequel l'attribut moléculaire est détecté par chromatographie d'exclusion stérique, chromatographie liquide haute performance (SEC-HPLC) et un compteur de particules de haute précision (HIAC).
